# EUROPEAN PATENT APPLICATION

(11) **EP 2 080 521 A1**
(43) Date of publication of application: **22.07.2009**
(21) Application number: 08021849.8
(22) Date of filing: 22.10.2003
(51) Int. Cl.: A61K 39/385, C07K 14/595, A61K 38/00

(54) **Gastrin compositions and formulations, and methods of use and preparation**

(30) Priority: 22.10.2002 US 420187 P; 22.10.2002 US 420399 P; 21.11.2002 US 428100 P; 22.11.2002 US 428562 P
(62) Divisional of application: 03774936.3
(71) Applicant: Waratah Pharmaceuticals, Inc., Toronto, ON M5G 1L7 (CA)
(72) Inventor: Brand, Stephen J., Lincoln, MA 01773 (US); Cruz, Antonio, Toronto ON M5P 2T7 (CA); Pastrak, Aleksandra, Toronto ON M4G 2V9 (CA); Hew, Yin, Thornhill ON L4J 5E7 (CA)
(74) Representative: Holliday, Louise Caroline

(57) **Abstract**

Compositions and methods are provided for islet neogenesis therapy comprising a member of a group of factors that complement a gastin/CCK receptor ligand, with formulations, devices and methods for sustained release delivery and for local delivery to target organs.

## Description

### Field of the Invention

The invention relates to methods and compositions for treating a diabetic subject with islet neogenesis therapy and an agent for immunosuppression, with formulations and methods for local delivery and for sustained release of the compositions.

### Background of the Invention

The severe forms of the common disease Diabetes Mellitus result from an absence or relative deficiency of insulin secretion from the pancreatic β cell. Consequently, diabetics are dependent on exogenous insulin injections to prevent life threatening complications of high blood glucose (hyperglycemia). Unless patients adhere to a very demanding regime of glucose testing and insulin treatment (intensive insulin treatment), insulin treatment does not prevent chronic long-term complications of organ damage caused by hyperglycemia. Intensive insulin treatment increases risk of acute hypoglycemia due to insulin overdosing, with acute and serious alterations of consciousness state that can be fatal.

About one million people in the United States population suffer from juvenile or type I diabetes, and about 30,000 new cases arise each year. Further, an extremely large and rapidly increasing number of patients have forms of type II diabetes (also called adult onset or insulin-resistance diabetes), at a level of epidemic proportions, a disease that can cause pancreatic exhaustion and insulin insufficiency.

The abnormally high blood glucose (hyperglycemia) that characterizes diabetes, if left untreated, results in a variety of pathological conditions, for example, non-healing peripheral vascular ulcers, retinal damage leading to blindness, and kidney failure. Diabetes of both types I and II are treated with insulin injection in response to blood glucose levels determined by patient glucose self-monitoring, although not all type II-patients progress to requiring insulin therapy. Typically, multiple doses of insulin are delivered by the patient per day. Severe pathological consequences of diabetes are correlated with less rigorous control of blood glucose level.

A potential treatment for diabetes would be to restore β cell function so that insulin release is dynamically regulated in response to changes in blood glucose levels. This can be achieved by pancreas transplantation, but this approach is typically limited to diabetics requiring kidney transplants for renal failure. Also, pancreas transplantation can require lifelong immunosuppression to prevent allogeneic graft rejection and autoimmune destruction of the transplanted pancreas.

Recently, transplants of isolated human islet preparations have successfully reversed insulin diabetes in human subjects for prolonged periods. However, a large amount of donor islet cell material is required for each recipient, and the supply of islet cell material has not been sufficient to meet the demand.

### Summary

A feature of the invention is a method for treating a diabetic condition, the method including administering to a mammal a therapeutically effective amount of a composition comprising a gastrin /CCK receptor ligand and a factor for complementing gastrin for islet neogenesis therapy (a FACGINT), provided that the FACGINT is not an EGF receptor ligand,

As the term is used herein, the FACGINT is at least one selected from the group of: a Glucagon-like peptide 1 receptor ligand; a Glucagon-like peptide 2 receptor ligand; a gastric inhibitory polypeptide (GIP) receptor ligand; a keratinocyte growth factor (KGF) receptor ligand; a dipeptidyl peptidase IV inhibitor; a REG protein receptor ligand; a Growth Hormone receptor ligand; a Prolactin (PRL) receptor ligand ; an Insulin-like Growth Factor (IGF) receptor ligand; PTH-related protein (PTHrP) receptor ligand; hepatocyte growth factor (HGF) receptor ligand; a bone morphogenetic protein (BMP) receptor ligand, a transforming growth factor-β (TGF-β) receptor ligand; a laminin receptor ligand; vasoactive intestinal peptide (VIP) receptor ligand; a fibroblast growth factor (FGF) receptor ligand; a keratinocyte growth factor receptor ligand; a nerve growth factor (NGF) receptor ligand; an islet neogenesis associated protein (INGAP) receptor ligand; an Activin-A receptor ligand; a vascular endothelial growth factor (VEGF) receptor ligand; an erythropoietin (EPO) receptor ligand; a pituitary adenylate cyclase activating polypeptide (PACAP) receptor ligand; a granulocyte colony stimulating factor (G-CSF) receptor ligand; a granulocyte-macrophage colony stimulating factor (GM-CSF); a platelet-derived growth factor (PDGF) receptor ligand and a Secretin receptor ligand.

As used herein, the term "diabetes" means any physiologic indication of a short age of insulin, a production of antibodies against insulin, or an excess of blood sugar. Diabetes is exemplified but not limited to diabetes I, diabetes II, gestational diabetes, and a pre-diabetic condition. As used herein, the term "mammal" has the usual meaning of any member of Mammalia, and includes humans.

In related embodiments, the FACGINT is a Glucagon-like peptide 1 (GLP-1) receptor ligand; a Glucagon-like peptide 2 (GLP-2) receptor ligand; or a member of a Growth Hormone receptor ligand family, and can be a Growth Hormone, such as Human Growth Hormone (HGH), a Placental lactogen (PL), or a Prolactin (PRL), or an exendin such as exendin-4.

Another feature of the invention provides a method for treating diabetes, the method including contacting *ex vivo* a plurality of cells with a composition having at least one of a FACGINT and a gastrin/CCK receptor ligand, provided that the FACGINT is not an EGF receptor ligand; and administering the cells to a mammal in need thereof, thereby treatingg the diabetes. In one embodiment of the method the cells are autologous, i.e., from the subject. Alternatively, the cells are from another individual in the same species, or even from another species. In the method using cells *ex vivo,* the cells can be pancreatic ductal cells. Pancreatic cells are a source of islet precursor cells. A further embodiment of this method involves, prior to the implanting step, treating the cells *ex vivo* with the composition. A related method further includes, prior to the contacting step, culturing the cells *ex vivo.*

Generally, the terms "administering" or "contacting" mean that the user of the method is provided the composition in an amount effective to increase the amount of insulin secreting cells in the mammal.

Generally in these methods, the composition is administered systemically. Further, the amount of the FACGINT in the composition is substantially less than the minimum effective dose of the FACGINT required to reduce blood glucose in the diabetic mammal in the absence of a gastrin/CCK receptor ligand. The method can further include measuring a parameter selected from the group of: blood glucose, serum glucose, blood glycosylated hemoglobin, pancreatic β cell mass, serum insulin, pancreatic insulin content, and morphometrically determined β cell mass. In general, one of skill in the art of diagnosis of diabetes would measure a glucose level in blood or serum following a fast, i.e., a period of no feeding of the subject or patient, of duration typical of such a diagnosis. The standard measurement is an assay of fasting blood glucose, or FBG.

The *in vivo* methods herein can further include measuring a parameter selected from the group of: amount of insulin secreting cells, glucose responsiveness of insulating secreting cells, amount of proliferation of islet precursor cells, and amount of mature insulin secreting cells, these measurements being made in a mammal that is an experimental animal such as a genetically diabetic mouse (NOD mouse) or a rodent in which diabetes has been induced (with streptozoticin or STZ).

Another embodiment herein is a method for inducing pancreatic islet neogenesis in a mammal which is administering to the mammal a composition comprising a combination of a FACGINT and a gastrin /CCK receptor ligand provided that the FACGINT is not an EGF receptor ligand, in an amount sufficient to increase proliferation of islet precursor cells in pancreatic tissue, thereby inducing pancreatic islet neogenesis.

Yet another embodiment herein in a method for inducing pancreatic islet neogenesis in a mammal, the method comprising administering a composition comprising a combination of a FACGINT and a gastrin /CCK receptor ligand wherein the FACGINT is not an EGF receptor ligand, in an amount sufficient to increase the number of pancreatic insulin secreting β cells in the mammal.

Accordingly, an embodiment of the invention is a composition comprising a gastrin/CCK receptor ligand and a FACGINT, provided that the FACGINT is not an EGF receptor ligand. The composition in some embodiments is provided in a dosage effective for inducing proliferation of islet precursor cells into an increased amount of mature insulin secreting cells. Similarly, the composition in some embodiments is provided in a dosage effective for inducing differentiation of an islet precursor cell into a mature insulin secreting cell.

The composition can be provided in a pharmaceutically acceptable carrier.

Also provided herein is a kit for treatingg diabetes, containing a composition comprising a gastrin/CCK receptor ligand and a FACGINT, a container, and instructions for use, provided that the FACGINT is not an EGF receptor ligand. The bit composition can be provided in one or more unit doses. The composition of the kit further includes a pharmaceutically acceptable carrier.

Another feature of the invention herein is a method for expanding and differentiating stem cells into insulin secreting cells in a diabetic recipient of implanted cells, which includes implanting the stem cells in the recipient, and administering to the recipient a composition containing an effective dose of each of a gastrin/CCK receptor ligand and a FACGINT provided that the FACGINT is not an EGF receptor ligand. In this method, the cells can be obtained for example from a human or a porcine. Further, the implanted cells are obtained from pancreatic islets, umbilical chords, embryos, or stem cell lines. Alternatively, a method for expanding and differentiating stem cells in a diabetic recipient of the cells into insulin secreting cells, is:implanting the cells in the recipient; and administering a sustained release composition comprising an effective dose of each of a gastrin/CCK receptor ligand, and a FACGINT or an EGF receptor ligand.

In general according to these methods, the gastrin/CCK receptor ligand is gastrin. In related embodiments, the gastrin is gastrin-17, for example, the gastrin is human gastrin 1-17Leu 15.

Further, according to these methods, implanting the cells in the recipient is using a route such as injecting directly into an organ, and administering intravenously. Injecting the cells is delivering locally into an organ, for example, the pancreas, the kidney, and the liver. Further, injecting the cells is delivering to the portal vein percutaneously or transhepatically. In any of these methods, a catheter can be inserted into a vein or artery leading from or to the organ, using an imaging technology, such as ultrasound or MRI during the procedure.

Delivering the cells locally can be chosen from several technologies, including endoscopic retrograde cholangiopancreatography (ERCP); endoscopic ultrasound-guided fine needle delivery (EUS-FNAD); injection into a pancreatic artery; injection into a portal vein; intrapancreatic injection; and injection into an hepatic artery. In these technologies the user can be guided by one of several imaging technologies including ultrasound or MRI during the procedure.

Another feature of the invention provided is a method for reducing an amount of stem cells needed for transplantation to treat human diabetes, the method including administering to the recipient an effective dose of each of a gastrin/CCK receptor ligand and a FACGINT, the amount of cells being reduced in comparison to implanting cells in the absence of administering the effective dose to an otherwise identical recipient, provided that the FACGINT is not an EGF receptor ligand.

In a related embodiment of the invention, the invention provides a composition comprising a gastrin/CCK receptor ligand and at least one FACGINT, provided that the FACGINT is not an EGF receptor ligand. The composition is provided in a dosage that is effective for inducing differentiation of an islet precursor cell into a mature insulin secreting cells. The composition can further be provided in a pharmaceutically acceptable carrier.

A kit is provided for treating diabetes, the kit containing a composition comprising at least one FACGINT provided that the FACGINT is not an EGF receptor ligand, and a gastrin/CCK receptor ligand, a container, and instructions for use.

Any of the above methods can further include administering to the subject an agent for suppressing an immune response. In any of the above methods, the components of the combination can be delivered simultaneously, for example, within one hour or within one day, or can be delivered sequentially, for example, at an interval of greater than one day, greater than two or more days, greater than one week.

An exemplary agent for suppressing immune response is a drug, for example, is at least one of the group consisting of a rapamycin; a corticosteroid; an azathioprine; mycophenolate mofetil; a cyclosporine; a cyclophosphamide; a methotrexate; a 6-mercaptopurine; FK506; 15-deoxyspergualin; an FTY 720; a mitoxantrone; a 2-amino-1,3-propanediol; a 2-amino-2[2-(4-octylphenyl)ethyl]propane-1,3-diol hydrochloride; a 6-(3-dimethyl-aminopropionyl) forskolin; and a demethimmunomycin.

Alternatively, an exemplary agent for suppressing immune response is a protein, for example, the protein comprises an amino acid sequence of an antibody. Accordingly, the agent for suppressing immune response is at least one of: hul 124; BTI-322; allotrap-HLA-B270; OKT4A; Enlimomab; ABX-CBL; OKT3; ATGAM; basiliximab; daclizumab; thymoglobulin; ISAtx247; Medi-500; Medi-507; Alefacept; efalizumab; infliximab; and an interferon. The islet neogenesis therapy composition and the agent for suppressing immune response are administered sequentially or can be administered simultaneously.

In certain embodiments, at least one of the islet neogenesis therapy composition and the agent for suppressing immune response is administered systemically. For example, the islet neogenesis therapy composition is administered as a bolus. Thus, at least one of the islet neogenesis therapy composition and the agent for suppressing immune response is administered by a route that is intravenous, subcutaneous, intraperitoneal, or intramuscular. Further, in certain embodiments, the agent for suppressing immune response is administered orally. In general, the agent for suppressing immune response is at least one of FK506, rapamycin, and daclizumab. Further, according to embodiments of any of the methods herein other than those calling for measurement of certain experimental parameters, the subject can be a human.

Also featured herein is a kit for treatment of a diabetic subject, comprising a container, an immunosuppressive agent, and an INT composition comprising a FACGINT provided that the FACGINT is not an EGF receptor ligand.

Also provided here is a pharmaceutical composition for sustained release of an I.N.T.^{™} therapeutic composition, the composition comprising: a gastrin receptor ligand; and an EGF receptor ligand or a FACGINT; wherein at least one of the gastrin receptor ligand, or the EGF receptor ligand or FACGINT, is a sustained release formulation.
This composition can further comprising an agent for immune suppression. Exemplary embodiments of the sustained release formulation are pegylation of at least one of the components of the composition, and formulation of at least one component in a multivesicular lipid-based liposome. Examples of the EGF receptor ligand is selected from the group consisting of an EGF and a TGFα. Examples of the FACGINT is at least one selected from the group of: a Glucagon-like peptide 1 receptor ligand; a Glucagon-like peptide 2 receptor ligand; a gastric inhibitory polypeptide (GIP) receptor ligand; a keratinocyte growth factor (KGF) receptor ligand; a dipeptidyl peptidase IV inhibitor; a REG protein receptor ligand; a Growth Hormone receptor ligand; a Prolactin (PRL) receptor ligand ; an Insulin-like Growth Factor (IGF) receptor ligand; PTH-related protein (PTHrP) receptor ligand; hepatocyte growth factor (HGF) receptor ligand; a bone morphogenetic protein (BMP) receptor ligand, a transforming growth factor-β (TGF-β) receptor ligand; a laminin receptor ligand; vasoactive intestinal peptide (VIP) receptor ligand; a fibroblast growth factor (FGF) receptor ligand; a keratinocyte growth factor receptor ligand; a nerve growth factor (NGF) receptor ligand; an islet neogenesis associated protein (INGAP) receptor ligand; an Activin-A receptor ligand; a vascular endothelial growth factor (VEGF) receptor ligand; an erythropoietin (EPO) receptor ligand; a pituitary adenylate cyclase activating polypeptide (PACAP) receptor ligand; a granulocyte colony stimulating factor (G-CSF) receptor ligand; a granulocyte-macrophage colony stimulating factor (GM-CSF); a platelet-derived growth factor (PDGF) receptor ligand; and a Secretin receptor ligand. Alternatively, the EGF receptor ligand is a low molecular weight drug. The composition can be formulated for parenteral administration. Alternatively, the composition can be formulated for oral administration.
The composition can be formulated for a route of administration selected from the group consisting of subcutaneous, intraperitoneal, intravenous, and intramuscular injection.
In one embodiment, at least one of the gastrin receptor ligand, or the EGF receptor ligand or the FACGINT, is formulated for systemic administration.

Further, the above compositions can be formulated for local delivery, for example, the local delivery is targeted to the pancreas. Exemplary types of local delivery include endoscopic retrograde cholangiopancreatography (ERCP); endoscopic ultrasound-guided fine needle aspiration delivery (EUS-FNAD); injection into a pancreatic artery; injection into a portal vein; intrapancreatic injection; and injection into an hepatic artery. These can be combined with imaging technologies such as ultrasound and MRI, performed during the procedure.

The composition in some embodiments is formulated for a route of administration selected from the group consisting of transdermal and mucosal delivery. Any of these compositions can be formulated for delivery by a mechanical device, in combination with formulation for sustained release, or alternatively, use of the mechanical device to deliver the formulation over a sustained period of time..The device is, for example, a degradable implant; a transcutaneous patch; a catheter; an implantable pump; a percutaneous pump; an infusion pump; and an iontophoresis device.

The above compositions and pharmaceutical compositions can be formulated for a route of administration selected from the group consisting of: subcutaneous, intraperitoneal, intravenous, and intrapancreatic. For example, the intravenous route is into a portal vein. A device can be used, and the device can be a pump. With sustained release formulations as with some of the above methods and compositions, the administration can be local. For example, the local administration can be delivered by a route selected from the group of: endoscopic retrograde cholangiopancreatography (ERCP); endoscopic ultrasound-guided fine needle aspiration delivery (EUS-FNAD); injection into a pancreatic artery; injection into a portal vein; intrapancreatic injection; and injection into an hepatic artery Alternatively with the sustained release formulations and devices, the administration can be systemic. The pharmaceutical composition can be provided in an effective dose. Also featured herein is a kit comprising at least one dose of a composition of any of the above sustained release formulations.

Also featured is a method of reducing frequency of treating a diabetic subject with an LN.T.^{™} composition, the method including preparing at least one component of the composition as a sustained release formulation; and administering the composition to the subject according to a protocol having greater intervals between treatments than for the composition not so formulated and otherwise identical. Administering can be delivering by a route selected from: endoscopic retrograde cholangiopancreatography (ERCP); endoscopic ultrasound-guided fine needle aspiration delivery (EUS-FNAD; injection into a pancreatic artery; injection into a portal vein; intrapancreatic injection; or injection into an hepatic artery. Imaging technology can be used to guide a catheter in place during these procedures.

Also featured is method of enhancing efficacy of an I.N.T.^{™} composition in a diabetic subject, the method including: administering to the subject an LN.T.^{™} composition having at least one component of the composition formulated to produce a sustained release; and comparing efficacy in treating the subject of an amount of the composition administered to efficacy of a composition not having a component so formulated and otherwise identical, such that the efficacy of the I.N.T.^{™} composition having a sustained release formulated composition, as measured by a decrease in an amount of the sustained release formulated agent required to reduce or eliminate symptoms of diabetes in the subject, is enhanced. In an embodiment of this methods, comparing efficacy is further analyzing toxicity of the composition, such that fewer or milder unwanted symptoms following administering the composition indicates decreased toxicity in the composition having at least one component formulated to produce a sustained release, compared to toxicity of the I.N.T.^{™} composition not having a component so formulated and otherwise identical. In any of these methods, exemplary sustained release formulations of the component are pegylation and a multivesicular lipid-based liposome.

In any of these methods enhancing efficacy of an I.N.T.^{™} composition, the component having the sustained release formulation is an EGF receptor ligand selected from the group consisting of an EGF and a TGFα. Alternatively in embodiments of these methods, the FACGINT is at least one selected from the group of: a Glucagon-like peptide 1 receptor ligand; a Glucagon-like peptide 2 receptor ligand; a gastric inhibitory polypeptide (GIP) receptor ligand; a keratinocyte growth factor (KGF) receptor ligand; a dipeptidyl peptidase IV inhibitor; a REG protein receptor ligand; a Growth Hormone receptor ligand; a Prolactin (PRL) receptor ligand ; an Insulin-like Growth Factor (IGF) receptor ligand; PTH-related protein (PTHrP) receptor ligand; hepatocyte growth factor (HGF) receptor ligand; a bone morphogenetic protein (BMP) receptor ligand, a transforming growth factor-β (TGF-β) receptor ligand; a laminin receptor ligand; vasoactive intestinal peptide (VIP) receptor ligand; a fibroblast growth factor (FGF) receptor ligand; a keratinocyte growth factor receptor ligand; a nerve growth factor (NGF) receptor ligand; an islet neogenesis associated protein (INGAP) receptor ligand; an Activin-A receptor ligand; a vascular endothelial growth factor (VEGF) receptor ligand; an erythropoietin (EPO) receptor ligand; a pituitary adenylate cyclase activating polypeptide (PACAP) receptor ligand; a granulocyte colony stimulating factor (G-CSF) receptor ligand; a granulocyte-macrophage colony stimulating factor (GM-CSF); a platelet-derived growth factor (PDGF) receptor ligand; and a Secretin receptor ligand. Even further as an alternative, a component is a low molecular weight drug. In any of these methods, administering is delivering by a route selected from the group consisting of parenteral, oral, transdermal, subcutaneous, mucosal, intraperitoneal, intravenous, intrapancreatic and intramuscular.

For example, administering produces local distribution. Further, the method includes administering the composition in an effective dose. Even further, the method includes, prior to administering, the composition is formulated for using a sustained release device. For example, the device selected from the group of: degradable implant; transcutaneous patch; catheter; implantable pump; percutaneous pump; infusion pump; and iontophoresis device. For example, the device is a pump. Administering in any of these methods may be by the intravenous route is injecting into a portal vein.

Also featured is a method of reducing frequency of treating a diabetic subject, the method comprising preparing a device for administering an LN.T.^{™} composition to the subject by continuous release for a prolonged period; providing the device to the subject; and re-iterating treating the subject by replacing or refilling the device, for example, a device which is a pump. The pump can be a percutaneous pump; a flurorcarbon propellant pump; an osmotic pump; a mini-osmotic pump; an implantable pump; or an infusion pump. Alternatively, the device is selected from the group consisting of: a degradable implant; a non-degradable implant; a mucoadhesive implant; a transcutaneous patch; a catheter; and an iontophoresis device. An exemplary non-degradable implant is Silastic. Other examples of degradable implants can be at least one of the materials selected from the group of: starch; vinylstarch; dipropyleneglycol diacrylate (DPGDA); tripropyleneglycol diacrylate (TPGDA); pectin; cellulose acetate; cellulose propionate; cellulose acetate butyrate; cellulose acetate propionate (CAP); hydroxypropyl cellulose (HPC); hydroxypropyl cellulose/cellulose acetate propionate (HPC/CAP); methyl methacrylate (MMA); butyl methacrylate (BMA); hydroxymethyl methacrylate (HEMA); ethyl hexyl acrylate (EHA); octadecyl methacrylate (ODMA); and ethyleneglycol dimethacrylate (EGDMA).

Also featured is a method for expanding and differentiating stem cells into insulin secreting cells in a diabetic recipient of the cells, having steps of: implanting the cells in the recipient; and administering a sustained release composition comprising an effective dose of each of: a gastrin/CCK receptor ligand; and a FACGINT or an EGF receptor ligand, wherein the stem cells are expanded and differentiated into insulin secreting cells in the recipient.

Also featured is a composition for treating diabetes comprising a Glucagon-like peptide-1 (GLP-1) receptor ligand and a gastrin/CCK receptor ligand. For example, the GLP-1 receptor ligand is GLP-1. Also featured is a composition for treating diabetes comprising a Growth Hormone (GH) receptor ligand and a gastrin/CCK receptor ligand. For example, the GH is human growth hormone (HGH). Also featured is a composition for treating diabetes comprising a prolactin (PL) receptor ligand and a gastrin/CCK receptor ligand. For example, the PL is human PL. In any of these compositions, the exemplary gastrin is gastrin I having 17 amino acids with a Leu residue at amino acid position 15. Any of these compositions can further have an agent for immune suppression. Any of these compositions can further be formulated for sustained release.

Also featured is a method of treating a diabetic subject, which is administering to the subject a composition comprising a gastrin /CCK receptor ligand and a Glucagon-like peptide-1 (GLP-1) receptor ligand. Also featured is a method of treating a diabetic subject which is administering to the subject a composition comprising a gastrin /CCK receptor ligand and a Growth Hormone (GH) receptor ligand. Also featured is a method of treating a diabetic subject which is administering to the subject a composition comprising a gastrin /CCK receptor ligand and a prolactin (PL) receptor ligand. Any of these methods can include administering an agent for immune suppression. Any of these methods can further include administering at least one of the receptor ligands or agents using a sustained release device. Any of these methods can include further include formulating at least one of the receptor ligands or agents for sustained release. Any of these methods can be used with the diabetic subject who has type I diabetes or type II diabetes.

Also featured is a method for expanding a functional β cell mass of pancreatic islet transplants in a diabetic patient recipient of a transplant of purified islets, the method being administering to the mammal an effective dose of a gastrinCCK receptor ligand and a FACGINT.

Also featured is a method for method of treating human diabetes comprising transplanting a pancreatic islet preparation into a diabetic patient; and administering to the patient an effective dose of a gastrin/CCK receptor ligand and a FACGINT. Accordingly in this method, the FACGINT comprises a prolactin receptor ligand which is prolactin.

### Detailed Description of Specific Embodiments

Compositions for islet neogenesis therapy (I.N.T.™) comprise a gastrin/CCK receptor ligand, and an EGF receptor ligand or a Factor Complementing Gastrin Islet Neogenesis Therapy (FACGINT).

Treatment of diabetes by administration with a combination of a FACGINT and a gastrin/CCK receptor ligand, for example, gastrin, gives a surprising level of enhancement of potency, efficacy and utility over treatment with the any single component alone. For this reason, the term FACGINT as used herein means "complementary" to administration of gastrin. The term "complementary" is not necessarily meant to imply a synergism between gastrin and the FACGINT, rather the term means that in comparison to administration of gastrin and the FACGINT, administration of the combination is more efficacious for remediating the diabetes at the doses used in the combination.

The term, "receptor ligand" as used herein in connection with a receptor for a particular ligand shall mean any composition or compound that binds to, interacts with, or stimulates that receptor.

The term "FACGINT" includes a large variety of growth factors and growth hormones, agents that modify one or more of the factors hormones, and ligands and effectors for one or more receptors involved in binding of these growth hormones and growth factors as these terms are generally understood, exemplified but not limited to: a PTH-related protein (PTHrP) receptor ligand such as PTHrP (PTHrP; Garcia-Ocana, A. et al., 2001, J. clin. Endocrin. Metab. 86: 984-988); a hepatocyte growth factor (HGF) receptor ligand such as HGF (HGF; Nielsen, J. et al., 1999, J Mol Med 77: 62-66); a fibroblast growth factor (FGF) such as FGF, a keratinocyte growth factor (KGF) receptor ligand such as KGF; a nerve growth factor (NGF) receptor ligand such as NGF; a gastric inhibitory polypeptide (GIP) receptor such as GIP; a transforming growth factor beta (TGFβ) receptor ligand such as TGFβ (U.S. patent application 2002/0072115 published Jun. 13, 2002), a laminin receptor ligand such as laminin-1; an islet neogenesis associated protein (INGAP) receptor ligand such as INGAP; a bone morphogenetic factor (BMP) receptor ligand such as BMP-2; a vasoactive intestinal peptide (VIP) receptor ligand such as VIP; a glucagon-like peptide 1 receptor ligand such as GLP-1 and exendin-4, glucagon-like peptide 2 (GLP-2) receptor ligand such as GLP-2, and dipeptidyl peptidase IV inhibitors which indirectly affect the levels of GLP-1 (Hughes, T. et al., 2002, Am Diabetes Assoc Abstract 272-or) by inhibiting an enzyme involved in its integrity; a REG receptor ligand such as REG protein; a Growth hormone (GH) receptor ligand such a GH, a Prolactin (PRL) receptor ligand such as PRL and placental lactogen (PL); an Insulin-like growth factor (Type 1 and 2) receptor ligands such as IGF1 and IGF-2; an Erythropoietin (EPO) receptor ligand such as EPO (http://www.drinet.org/html/august_2002_.htm); a betacellulin (also considered to be a member of the EGF family); an Activin-A receptor ligand such as Activin-A; a vascular endothelial growth factor (VEGF) receptor ligand such as VEGF; a bone morphogenesis factor (BMP) receptor ligand such as BMP-2; a vasoactive intestinal peptide (VIP) receptor ligand such as VIP; a vascular endothelial growth factor (VEGF) receptor ligand such as VEGF; a pituitary adenylate cyclase activating polypeptide (PACAP) receptor ligand such as PACAP; a granulocyte colony stimulating factor (G-CSF) receptor ligand such as G-CSF; a granulocyte-macrophage colony stimulating factor (GM-CSF) receptor ligand such as GM-CSH; a platelet-derived growth factor (PDGF) receptor ligand such as PDGF and a Secretin receptor ligand such as secretin.

For any of the growth factors, enzymes, enzyme inhibitors, peptide, protein and hormone compounds herein that are indicated to be an exemplary FACGINT, all known analogues, variants, and derivatives, whether naturally occurring or made by mutagenesis or designed and synthesized shall be considered equivalent to that FACGINT. Also considered among equivalents are conjugates, i.e., compositions derived by addition of one or more of a chemical group, and mixtures thereof. Encoding genes may be altered by, for example, oligonucleotide directed mutagenesis to produce FACGINT analogues thereof, such as the human recombinant analogues. Further, an identity or location of one or more than one amino acid residue may be changed by targeted mutagenesis. The primary amino acid sequence of the protein may be augmented by conjugates, as by glycosylation, acylation, or by addition of any other supplementary molecules, such as one or more of a lipid, a phosphate, and/or an acetyl group. Further, individual amino acid residues in the chain may be modified by oxidation, reduction, or other derivatization. The FACGINT may be cleaved to obtain any fragments which retain activity. An agonist, a prodrug or a metabolite of a FACGINT is equivalent to that FACGINT. The whole polypeptide or protein or any fragment can be fused with any other peptide or protein such as immunoglobulins and other cytokines. Variants of FACGINTs that are proteinaceous in nature can result from alternative splicing of a primary transcript, from proteolytic cleavage or from other post-translational modifications including dimerization, polymerization, phosphorylation, glycosylation, sulfation and deamidation. Conjugates may include, for example, a composition comprising the FACGINT coupled to a non-naturally occurring polymer comprising a polyalkylene glycol moiety. The term also encompasses derivatives obtained by chemically modifying one or more amino acid residues of the parent peptide, for instance by alkylation, acylation, ester formation or amide formation. Further, agents that induce synthesis of the FACGINT or mimic the action of the FACGINT are contemplated as equivalent compounds. The singular form, "FACGINT", may mean any one or more compounds from the exemplary FACGINTs shown herein.

In several uses of the term herein, the term FACGINT is specified not to include an EGF receptor ligand, as is made plain in the context. However EGF receptor ligands such as EGF and TGF are capable of complementing gastrin for remediation of diabetic conditions and are therefore exemplary FACGINTs as defined herein, and are included as components in other embodiments of compositions, methods and kits herein. Certain embodiments of LN.T. therapy comprising administration of an EGF receptor ligand in combination with a gastrin/CCK receptor ligand have been described previously (U.S.patent numbers 5,885,956 and 6,288,301), references that do not use these agents in certain combinations and formulations as shown herein.

The term, "pancreatic progenitor cell" or "beta cell progenitor" or "islet precursor cell" as used herein is a precursor cell capable of differentiating into a pancreatic beta islet cell, which may or may not have the characteristic of a stem cell that is the ability to reproduce itself in an unlimited manner. "Beta cell neogenesis" or "islet neogenesis" means formation of new beta cells by differentiation, which may or may not have the characteristic of a stem cell that is the ability to reproduce itself in an unlimited manner. To secrete insulin in a "glucose-responsive" manner means to secrete insulin according to the glucose concentration in the blood. In a physiologically normal mammal, beta cells of the islets of Langerhans secrete insulin as blood glucose level is elevated, i.e., are induced to secrete insulin in response to blood glucose level.

A receptor "agonist" is any composition without limitation, for example, a polypeptide growth factor or cytokine that is endogenously found in the mammal, or a variant or portion thereof, or an analog, or any peptidomimetic or low molecular weight drug, that has the capability to bind to and activate the receptor of the endogenously found polypeptide factor. For any of the growth factors or cytokines described herein, an equivalent is considered one that is substantially identical in amino acid sequence, for example, shares 50% sequence identity, shares 60% sequence identity, shares 70% sequence identity, or shares 80% sequence identity with a naturally occurring peptide or protein as described herein. In other embodiments, the agonist compositions herein include an agonist inducing agent, which are envisioned to be substances that, when given to an animal or provided to a cell, organ or tissue in culture, is capable of increasing the amount of that agonist produced by the animal, cell, organ or tissue. For example, a prolactin release peptide stimulates the secretion of prolactin. A receptor ligand includes within the scope of the definition a receptor agonist, for the receptor for any particular FACGINT, whether or not the agonist is structurally related to the FACGINT.

The invention in one embodiment provides a method for treating diabetic conditions such as diabetes mellitus by administering a composition comprising both a gastrin/CCK receptor ligand, e.g. gastrin, and a FACGINT, e.g. GLP-1, PRL or GH. Without being limited by any particular mechanism, the gastrin/CCK receptor ligand and the FACGINT are provided in an amount of each sufficient to effect differentiation of pancreatic islet precursor cells to mature insulin-secreting cells. Each of the FACGINT and the gastrin/CCK receptor ligand in the composition can be administered systemically or locally. Alternatively, one or both of the FACGINT and the gastrin/CCK receptor ligand can be expressed *in situ*, by cells that have been provided with a nucleic acid fusion construct in an expression vector. The fusion construct typically includes a preprogastrin peptide precursor coding sequence, and also a coding sequence for a FACGINT.

Administration of a gastrin/CCK receptor ligand and an EGF receptor ligand can achieve prolonged efficacy of islet cell neogenesis, such that a therapeutic benefit is retained long after cessation of treatment. See PCT application PCT/US02/00685 (WO 02/055152), published 18 July 2002. The duration of the therapeutic benefit is greater than the duration of the protocol for administration of the composition.

Regenerative differentiation of residual pluripotent pancreatic ductal cells into mature insulin-secreting cells is obtained with the provided compositions and methods for treatment of diabetes mellitus, particularly juvenile onset diabetes, and by therapeutic administration of this combination of factors or compositions which are provided for systemic administration, or for *in situ* expression within the pancreas.
An approach to β cell replacement that eliminates a requirement for cell transplantation is stimulation of β cell regeneration. Although early studies suggested that a β cell has limited capacity for regeneration, it has been increasingly realized that the insulin secreting β cells of the pancreas comprise a dynamic cell population. The mass of β celts can expand through proliferation of existing β cells β cell replication). During pregnancy, prolactin, growth hormone (Holstad, M. et al., J. Endocrinol. 163:229-234), and placental lactogen (Nielsen, J.H., et al., J. Mol. Med. 77:62-66, 1999) stimulate the proliferation of β cells to increase β cell mass. However, this expanded mass of cells depends on continuing hormonal stimulation. After parturition, the expanded β cell mass decreases to non-pregnant levels, in response to the decrease in prolactin and placental lactogen (Logothetopoulos, J., (Logothetopoulos, J. (1972) in Handbook of Physiology (Am. Physiol. Soc., Washington, DC), Section 7, Chapter 3, pp67-76).

From this physiological information, an important aspect in evaluating β cell regeneration in response to administration of a gastrin receptor ligand and either an EGF receptor ligand or a FACGINT is whether an expanded β cell mass can persist for a significant time after the cessation of treatment with growth factors. Use of a sustained release formulation, in combination with the I.N.T. composition, with or without an agent for immune suppression, can avoid a requirement for frequent visits to a medical setting, or for self-medication.

The neogenesis of β cells is measured as an increase in both cell number and cell mass, resulting in an increase in plasma insulin levels or pancreatic insulin content. Prolonged efficacy in treatment of diabetes is a desired outcome of I.N.T.

An embodiment of the present invention provides improved methods and compositions for use of a FACGINT administered with a gastrin/CCK ligand to treat diabetes. The present invention in one embodiment provides a combination of gastrin with a FACGINT to achieve greater efficacy, potency, and utility than achieved with a component alone, resulting in an improved therapeutic ratio for the combination. Treatment with a combination of gastrin and a FACGINT gave a reduction in blood glucose that was greater than observed after treatment with a component alone, and the reduction in blood glucose was sustained for a prolonged period after ceasing treatment. The phrase, "a FACGINT" as used herein can also mean "one or more FACGINTs" or "at least one FACGINT".

The β cell stimulant Exendin (a GLP-1 analog) improved glucose tolerance and increased β cell mass in a partial pancreatectomy model with mild diabetes (Xu, G. et al., Diabetes 48:2270-2276, 1999). However, a causal relationship between the improved glucose tolerance and β cell regeneration was not conclusively established. GLP-1, as shown here to be an exemplary FACGINT, when administered alone suppresses appetite and enhances the clearance of glucose by reducing insulin resistance, a process which can be independent of β cell stimulation. Thus the finding that plasma insulin levels were lower, not higher, in an Exendin treated group, suggests that the observed improved glucose tolerance was not a result of β cell stimulation. Furthermore, evaluation of the effect of Exendin on β cell growth is complicated by the pancreatectomy model of diabetes that was used in these studies. An inflammation resulting from the surgical ablation of the pancreas causes expression of growth factors that act on islets such as gastrin and TGFα, which by themselves stimulate islet regeneration. Indeed, enhanced β cell regeneration has been reported after pancreatectomy alone (Bonner-Weir, S., Diabetes 42: 1715-1720, 1993; Sharma, A., et al., Diabetes 48:507-513, 1999). Thus, it has not previously been clear that Exendin could stimulate enhanced β cell regeneration in the absence of these pancreatectomy-induced growth factors.

The term, "diabetes" as used herein means any manifested symptoms of diabetes in any mammal including experimental animal models, and including human forms such as type I and type II diabetes, early stage diabetes, and a pre-diabetic condition characterized by mildly decreased insulin or mildly elevated blood glucose levels. A "pre-diabetic condition" describes a mammal suspected of having a diabetic or related condition, for example, not formally diagnosed with diabetes, but demonstrating a symptom in terms of insulin or glucose level, and susceptibilty to diabetes or a related condition due to family history, genetic predisposition, or obesity in the case of type II diabetes, or has previously had diabetes or a related condition and is subject to risk of recurrence.

As used herein, the term "gastrin/CCK receptor ligand" encompasses any compound that binds to, interacts with or stimulates the gastrin/CCK receptor. Examples of such gastrin/CCK receptor ligands are given in U.S. patent 6,288,301 issued Sept. 11, 2001, and include various forms of gastrin, such as gastrin 34 (big gastrin), gastrin 17 (little gastrin), and gastrin 8 (mini gastrin); various forms of cholecystokinin such as CCK 58, CCK 33, CCK 22, CCK 12 and CCK 8; and other gastrin/CCK receptor ligands. In general, gastrin/CCK receptor ligands share a carboxy terminal amino acid sequence Trp-Met-Asp-Phe-amide. Also contemplated are active analogs, fragments and other modifications of the above, including both peptide and non-peptide agonists or partial agonists of the gastrin/CCK receptor such as A71378 (Lin et al., Am. J. Physiol. 258 (4 Pt 1): G648, 1990).

Small forms of gastrin such as gastrin 17 are economically prepared by peptide synthesis, and synthetic peptides are commercially available. Synthetic human gastrin 17 such as human gastrin 17 having methionine or leucine at position 15 are also available from Bachem AG, Bubendorf, Switzerland, and from Researchplus. Gastrin/CCK receptor ligands include also active analogs, fragments and other modifications of the above ligands, which for example share amino acid sequence with an endogenous mammalian gastrin, for example, share 60% sequence identity, or 70% identity, or 80% identity. Such ligands also include compounds that increase the secretion of endogenous gastrins, cholecystokinins or similarly active peptides from sites of tissue storage. Examples of these are the gastric releasing peptide, omeprazole which inhibits gastric acid secretion, and soya bean trypsin inhibitor which increases CCK stimulation.

The method for treating diabetes mellitus in an individual in need thereof includes administering to the individual a composition that provides both a gastrin/CCK receptor ligand and a FACGINT. Without being limited by any particular mechanism, the gastrin/CCK receptor ligand and the FACGINT are provided in doses sufficient to effect differentiation of pancreatic islet precursor cells to mature insulin-secreting cells.

The term "treating" or "ameliorating" as used herein means reducing or eliminating one or more symptoms of a diabetes. A method provided herein for treating diabetes comprises administering, without being limited by the specific mechanism, a differentiation regenerative amount of both a gastrin/CCK receptor ligand and a FACGINT, to a diabetic mammal, to stimulate islet neogenesis to increase the number of functional glucose responsive insulin secreting β cells in the pancreas. The method is effective for diabetes generally, including Type I or juvenile diabetes mellitus. The combination of gastrin and FACGINT would result in a significant enhancement of the islet neogenesis response over that observed with the individual components. An exemplary gastrin/CCK receptor ligand is gastrin, and exemplary FACGINTs are GLP-1, PRL and GH.

Another embodiment of the invention herein is a method comprising treating explanted pancreatic tissue of a mammal with a gastrin/CCK receptor ligand and FACGINT *ex vivo,* and introducing the treated pancreatic tissue to the mammal. Again in this method, an exemplary gastrin/CCK receptor ligand is gastrin, and exemplary FACGINTs are GLP-1, PRL and GH.

In another embodiment, the invention provides a method for gastrin/CCK receptor ligand stimulation, the method comprising providing a chimeric insulin promoter-gastrin fusion gene to pancreatic cells and expressing the gene. In yet another embodiment, a method of FACGINT stimulation is provided, comprising expressing a FACGINT gene that was trangenically introduced into a mammal, for example, a gene encoding a FACGINT, for example, GLP-1, PRL or GH. The gastrin/CCK receptor ligand gene can similarly be provided trangenically, preferably a human preprogastrin peptide precursor gene as shown in U.S. patent number 5,885,956.

As used herein the term mammal shall include without limitation any members of the Mammalia, such as a human, an ape, a rodent such as a mouse or rat, a dog, a cat, an agriculturally important animal or a protein pig, a goat, a sheep, a horse, or an ape such as a gorilla or a chimpanzee. An individual mammal may be non-diabetic, pre-diabetic, or diabetic, as specified herein.

Modes of systemic administration include, but are not limited to, transdermal, intrathecal, intramuscular, intraperitoneal, intravenous, subcutaneous, intranasal, and oral routes. The compounds may be administered by any convenient route, for example, by infusion or bolus injection, by absorption through epithelial or mucocutaneous linings (e.g., oral mucosa, rectal, vaginal, nasal, and intestinal mucosa, etc.), and may be administered together with other biologically active agents. An exemplary route of administration is systemic, for example, by subcutaneous injection.

The term, "prolactin" as used herein means any polypeptide which shares substantial sequence similarity with an endogenous mammalian prolactin as this term is known in the art of protein factors, for example, human prolactin, and which possesses the activity of a prolactin. Endogenous human prolactin is a 199 amino acid polypeptide produced by the pituitary gland. The term encompasses prolactin analogs which are deletions, insertions, or substitution mutants of endogenous prolactin, and retain the activity, and includes prolactins from other species and naturally occurring variants. The prolactin function includes a composition having agonist activity for the prolactin receptor, as disclosed in U.S. patent number 6,333,031 (activating amino acid sequence) and 6,413,952 (metal complexed receptor ligand agonist), and G120RhGH, which is an analog of human growth hormone that acts as a prolactin agonist (Mode et al., 1966, Endocrinol. 137(2): 447-454), and a ligand for the prolactin receptor as described in U.S. patents 5,506,107 and 5,837,460. Also included are prolactin-related protein, S 179D, human prolactin and placental lactogens.

PRL, GH and PL are members of a family of polypeptide hormones that share a structural, immunological and biological functions (reviewed in, "Pancreatic Growth and Regeneration", Ed. N. Sarvetnick, Ch. 1. Brejie, T. et al., 1997), and therefore referred to herein as the PRL/GH/PL family. PRL and GH are secreted by the anterior pituitary of vertebrate animals. PRL is involved in a broad range of biological functions that include osmoregulation, reproduction, lactation, and immunomodulation. GH is associated with physiological processes related to growth and morphogenesis. The related receptor ligands are referred to as "PRL/GH/PL" receptor ligands. Classification of FACGINTs into various groups based on structural similarity of the peptides and proteins, functional similarity with respect to complementation of gastrin, functional similarity with respect to binding of one or more receptors, are each within the scope of various embodiments of the invention. Prolactin receptor ligands include PRL and PL, and growth hormone receptor ligands include GH.

As used herein, the term "GLP-1 receptor ligand" encompasses any compound that binds to, interacts with or stimulates the GLP-1 receptor. Examples of GLP-1 receptor ligand include GLP-1 and exendin-4. Glucagon-like peptide-1 is synthesized in intestinal endocrine cells in molecular forms GLP-1 (having residues conventionally designated as positions 7-36) which is an amide, and and similarly as GLP-1(7-37). Initial studies of GLP-1 biological activity in utilized the full length N-terminal extended forms of GLP-1 (1-37 and 1-36 which latter is an amide). The larger GLP-1 molecules were generally lacking biological activity. It was later found that removal of the first six amino acids resulted in a shorter version of the GLP-1 molecule having substantially enhanced biological activity.

The majority of circulating biologically active GLP-1 is found in the GLP-1 (7-36)amide form, with lesser amounts of the bioactive GLP-1(7-37) form also detectable. See Orskov, C. et al., Diabetes 1994, 43: 335-339. Both peptides show about the same amount of biological function. GLP-1 is secreted from gut endocrine cells in response to nutrient ingestion and plays multiple roles in metabolic homeostasis following nutrient absorption. Regulation of GLP-1 occurs by N-terminal degradation of the peptide by Dipeptidyl Peptidase (DPP-IV) -mediated cleavage at the position 2 alanine residue. For an overview, see DPP-IV. The biological activities of GLP-1 include stimulation of glucose-dependent insulin secretion and insulin biosynthesis, inhibition of glucagon secretion and gastric emptying, and inhibition of food intake. GLP-1 appears to have a number of additional effects in the GI tract and central nervous system, as reviewed in Drucker, D., Endocrin 142: 521-527, 2001. Exemplary GLP-1 compositions include: BIM 51077 (GLP-1 analog resistant to DPP-IV digestion, available from Beaufour Ipsen); AC2592 (GLP-1, from Amylin, San Diego CA); ThGLP-1 (GLP-1, modified amino acids and fatty acid attachment, from Theratechnologies, Saint-Laurent, Quebec, Canada); CJC-1131 also known as DAC™:GLP-1 (GLP-1 analog engineered for covalent coupling to albumin, Conjuchem, Montreal, Quebec, Canada), LY315902 and sustained release LY315902 (DDP-IV resistant GLP-1 analog from Eli Lilly, Indianapolis, IN); a low molecular weight GLP-1 mimetic; Albugon (albumin: GLP-1 fusion peptide from Human Genome Sciences, Rockville, MD); Liraglutide also known as NN2211 (long acting GLP-1 derivative that is obtained by acylation of the GLP-1 molecule, which upon entering the bloodstream, is extensively bound to albumin which protects it from degradation by DPP-IV and reduces renal clearance, available from Novo Nordisk, Denmark; Elbrond et al., Diabetes Care 2002 Aug 25(8): 1398-404).

Exendin-4, an example of an exendin, is a novel peptide from *Heloderma suspectum* (Gila monster) venom, having 53% homology with GLP-1 (7-36)amide. It functions as a long-acting potent agonist of the glucagon-like peptide 1 (GLP-1) receptor, as it is resistant to degradation by DDP-IV. Exendin-4 has properties similar to GLP-1, and regulates gastric emptying, insulin secretion, food intake, and slucagon secretion. Examples of exendin-4 include exenatide (synthetic form also known as AC2993, Amylin); exenatide LAR (longactinf form); ZP10 (modified exendin-4 having addition of six lysine residues, Aventis/Zealand Pharma); and AP10 (long acting formulation, Alkermes, Cambridge MA). Physiological studies indicate that sustained expression of exendin-4 in transgenic mammals does not perturb glucose homeostasis, cell mass or food intake (Biaggio, L. et al. J Biol Chem 275: 34472-34477, 2000), so that the physiological effects of exendin-4 are not completely understood.

Dipeptidyl peptidase IV (DPP-IV) inhibitors refer to compounds that inhibit activity of DPP-IV, a membrane-associated peptidase of 766 amino acids that includes in its substrates GLP-1, GLP-2 and GIP. DPP-IV-mediated inactivation of GLP-1 is a determinant of GLP-1 bioactivity in vivo. Examples of DPP-IV inhibitors include isoleucine thiazolidide, valine-purrolidide, NVP-DPP738 (Novartis, Cambridge, MA), LAF237 (Novartis), P32/98 (Probiodrug AG, Halle, Germany), and P93/01 (Probiodrug).

As used herein, the term "EGF receptor ligand" encompasses compounds that stimulate the EGF receptor such that when gastrin/CCK receptors in the same or adjacent tissue or in the same individual are also stimulated, neogenesis of insulin-producing pancreatic islet cells is induced. Examples of such EGF receptor ligands include full length EGF, which is EGF1-53, and further include EGF1-48, EGF1-49, EGF1-52, and fragments and active analogs thereof. Other examples of EGF receptor ligands are TGFα forms that include 1-48, 1-47, 1-51, and amphiregulin and pox virus growth factor as well as any EGF receptor ligands that demonstrate the same synergistic activity with gastrin/CCK receptor ligands. These include active analogs, fragments and modifications of the above. See also, Carpenter and Wahl, Chapter 4, in Peptide Growth Factors (Eds. Sporn and Roberts), Springer Verlag, 1990.

The group of compounds comprises the EGF receptor ligands further includes "modified EGF", which includes variants of normal or wild type EGF. Modifications have been shown to affect one or more biological activity such as the rate of clearance of EGF. The term includes peptides having an amino acid sequence substantially similar to that of human EGF, for example, with one or a few amino acid substitutions at various residue positions.

Recombinant EGF forms have been genetically engineered to have alterations in structure and activities, for example, EGF having a methionine at position 21 replaced by a leucine residue has been described (U.S. patent number 4,760,023). Recombinant human EGF (hEGF) having 51 residues, i.e., lacking the two C-terminal residues at positions 52 and 53 of hEGF, and having a neutral amino acid substitution at position 51, retain EGF activity and are more resistant to protease degradation during a microbial production process, and following administration to a subject. A series of nucleic acid molecules have been described that encode a family of proteins that have significant similarity to EGF and TGFα (WO 00/29438). EGF muteins (mutated EGF) having histidine at residue 16 replaced with a neutral or acidic amino acid have been described (WO 93/03757), such forms retaining activity at low values of pH. Chemical analogues and fragments of EGF and TGFα retain ability to bind various members of the EGF receptor family (U.S. patent number 4,686,283). Various modifications of EGF or TGFα confer advantageous properties affecting one or more of recombinant protein production, in vitro and in vivo stability, and in vivo activity. A exemplary recombinant modified EGF receptor ligand used in the Examples herein is a C-terminus deleted form of human EGF of 51 amino acids in length, having asparagine at position 51 (referred to herein as EGF51N), which retains substantially full I.N.T.^{™} activity, and has in vivo and/or in vitro stability that is that is at least about as great or greater than normal or wild type hEGF (S. Magil et al., published May 15, 2003 as PCT/US02/33907, and incorporated by reference herein in its entirety).

The term, "growth hormone" as used herein encompasses any polypeptide that shares substantial amino acid sequence identity with an endogenous mammalian growth hormone and possesses a biological activity of a mammalian growth hormone. Human growth hormone is a polypeptide containing 191 amino acids in a single chain, and a molecular weight of about 22 kDal (Goeddel et al., 1979, Nature 281: 544-548; Gray et al., 1985, Gene 39: 247-254). The term encompasses analogs having deletions, insertions or substitutions and growth hormones from other species and naturally occurring variants. See Cunningham et al., 1989, Science 243: 1330-1336, and 1989, Science 244: 1081-1085; and WO 90/05185, and U.S. patent number 5,506,107.

The term, "erythropoietin" (EPO) as used herein is any endogenous mammalian EPO or variant thereof, or EPO receptor agonist, for example the EPO mimetic EMP1 (Johnson et al., 2000, Nephr Dial Tranpl 15:1274-1277); or mimetics described (Wrighton et al., 1996, Science 273:458-464; U.S. patent number 5,773,569; Kaushansky, 2001, Ann NY Acad Sci 938: 131-138); an antibody having EPO receptor agonist activity (see for example, U.S. patent number 5,885,574; WO 96/40231); and amino acid sequence disclosed in U.S. patent number 6,333,031, and 6,413,952.

The term, "PACAP" as used herein means an endogenously produced PACAP or analog or variant thereof that shares substantial amino acid identity or similarity, or has biological activity as a PACAP receptor agonist such as maxadilan (Moro et al., 1997, J Biol Chem 272:966-970. Useful PACAP variants include without limitation, 38 amino acid and 27 amino acid variants as disclosed in U.S. patent numbers 5,128,242; 5,198,542; 5,208,320; and 6,242,563).

### Pharmaceutical Compositions

The present invention in various embodiments provides pharmaceutical compositions comprising a therapeutically effective amount of a combination of a FACGINT, and a gastrin/CCK receptor ligand. All of the pharmaceutical compositions described herein can be formulated with or without an agent for immune suppression, and with or without components or devices for sustained release, for delivery locally or systemically. A pharmaceutically acceptable carrier or excipient can be added. Such a carrier includes but is not limited to saline, buffered saline, dextrose, water, glycerol, ethanol, and combinations thereof. The formulation should suit the mode of administration. An "effective amount" as the term is used herein is an amount of a therapeutic agent or combination of agents sufficient to achieve a recognized medical endpoint, in this case, remediation of a symptom of diabetes. The effective amount can be determined empirically by a skilled artisan according to established methods of measurement of relevant parameters, as described herein.

The compositions herein can further comprise wetting or emulsifying agents, or pH buffering agents. The composition can be a liquid solution, suspension, emulsion, tablet, pill, capsule, sustained release formulation, or powder. The compositions can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Various delivery systems are known and can be used to administer a composition of the invention, e.g., encapsulation in liposomes, microparticles, microcapsules and the like.

In an exemplary embodiment, a composition herein is formulated in accordance with routine procedures as a pharmaceutical composition adapted, for example, for subcutaneous administration to human beings. Typically, compositions for subcutaneous administration are solutions in sterile isotonic aqueous buffer. Where necessary, the composition may also include a solubilizing agent and a local anesthetic to ameliorate pain at the site of the injection. Generally, the ingredients are provided either separately or mixed together in unit dosage form, for example, as a dry, lyophilized powder or water-free concentrate in a hermetically sealed container such as an ampoule or sachette, for example, indicating the quantity of active agent. Where the composition is to be administered by infusion, it can be dispensed with an infusion bottle containing sterile pharmaceutical grade water, buffer, or saline. Where the composition is administered by injection, an ampoule of sterile water or saline for injection can be provided so that the ingredients may be mixed prior to administration. The compositions herein can in various components thereof be formulated as suppositories, which contain active ingredient in the range of about 0.5% to about 10% by weight; oral formulations preferably contain about 10% to about 95% active ingredient by weight.

A daily dose is administered as a single dose, or is divided into a plurality of smaller fractional doses, to be administered several times during the day.

The compositions of the invention can be formulated as neutral or salt forms. Pharmaceutically acceptable salts include those formed with free amino groups such as those derived from hydrochloric, phosphoric, acetic, oxalic, tartaric acids, etc., and those formed with free carboxyl groups such as those derived from sodium, potassium, ammonium, calcium, ferric hydroxides, isopropylamine, triethylamine, 2-ethylamino ethanol, histidine, procaine, etc.

The amount of the therapeutic of the invention which will be effective in the treatment of a particular disorder or condition will depend on the nature of the disorder or condition, and can be determined by standard clinical techniques. The precise dose to be employed in the formulation will also depend on the route of administration, and the seriousness of the disease or disorder, and should be decided according to the judgment of the practitioner and each patient's circumstances. Routine determinations of blood levels of insulin or C peptide, and of fasting levels of glucose or glucose challenges, are determined by one of ordinary skill in the art. Effective doses may be extrapolated from dose-response curves derived from in vitro or animal model test systems, by one of ordinary skill in the art of pharmacology. Suitable dosage ranges for administration are generally about 0.01 micrograms to about 10,000 micrograms of each active I.N.T.^{™} compound per kilogram body weight per day, for example, about 0.01 micrograms to about 1 microgram/kg, about 0.1 micrograms/kg to about 10 micrograms/kg, about 1 microgram/kg to about 500 micrograms/kg, or about 10 micrograms/kg to about 10 mg/kg of body weight per day. Suitable dosage ranges for administration are thus generally about 0.01 micrograms/kg body weight/day to about 10 mg/kg body weight/day.

The invention in other embodiments provides a pharmaceutical pack or kit comprising one or more containers filled with one or more of the ingredients of the pharmaceutical compositions of the invention. In such a pack or kit can be found a container having a unit dosage of each or both of a gastrin/CCK receptor ligand and/or one or more of a FACGINT, or an EGF receptor ligand, and one or more of an immunosuppressive agent. One or more of these components of the pack or kit can be formulated for sustained release or for insertion as a refill into a sustained release device, or can be formulated for local delivery. Associated with such container(s) can be various written materials such as instructions for use, or a notice in the form prescribed by a governmental agency regulating the manufacture, use or sale of pharmaceuticals or biological products, which notice reflects approval by the agency of manufacture, use or sale for human administration. In some embodiments the kit or pack may be associated with software embedded in a computer readible format.

The invention in one aspect features islet neogenesis therapy (I.N.T.^{™}) compositions and methods, for example, a gastrin and a FACGINT, in combination with immunosuppressive agents, to stimulate the growth of new β cells in vivo, increasing islet mass, and result in improved glucose tolerance in diabetic subjects, for example, in diabetic humans and in animals.

The gastrin/CCK receptor ligand and the EGF receptor ligand or FACGINT, can be administered in a single combined dose, or administered separately in any order. An "effective combined dose" of these compositions is a dose that produces a decrease in fasting blood glucse, or an increase in amount of insulin secreting cells, or an increase in insulin blood level, or an increase in β-cell mass. The gastrin/CCK receptor ligand is, in one embodiment, human gastrin of length 17 amino acid residues, the residue at position 15 being leucine (1-17Leu15, referred to herein as gastrin17leu15); further, the EGF receptor ligand is human EGF51N (S. Magil et al., published May 12, 2003 as PCT/US02/33907, and incorporated by reference herein in its entirety). The effective dose can contain a ratio of gastrin/CCK receptor ligand to EGF receptor ligand that is greater than 1, for example, the effective dose contains a ratio of gastrin/CCK receptor ligand to EGF receptor ligand greater than 10. A convenient route of administering the dose is with a systemic injection, for example, a subcutaneous bolus.

In a further embodiment, the recipient subject is administered an agent that suppresses the immune system. For example, the agent is a low molecular weight organic chemical, for example, is at least one of Tacrolimus, Sirolimus, cyclosporine, and cortisone and other drugs as shown in Table 1. In an alternative embodiment, the agent is an antibody, for example, the antibody is anti-CD11a and other antibodies also shown in Table 1. In yet another alternative, the immunosuppressive agent can be an antibody that is elaborated by the subject following an immunization schedule, for example, against GFAP or against S100β. The subject can be diabetic, for example, the subject is a non-obese diabetic mouse (the NOD mouse) or a streptozoticin-treated mouse. The subject can be a human, for example a diabetic patient having type I or type II diabetes, or having a pre-diabetic condition, or having gestational diabetes, or having had diabetes in the past, for example, having had gestational diabetes in a past pregnancy.

Further, evaluating the size and function of newly developed β insulin secreting cells or islets is measuring a standard physiological or diagnostic parameter including: islet β cell mass, islet β cell number, islet β cell percent, blood glucose, serum glucose, blood glycosylated hemoglobin, pancreatic β cell mass, pancreatic β cell number, fasting plasma C peptide content, serum insulin, and pancreatic insulin content.

As diabetes is in certain cases an autoimmune disease, an embodiment of I.N.T.^{™} is systemic administration of therapeutically effective doses of, for example, the ligands of receptors for each of EGF and gastrin/CCK or each of a FACGINT and gastrin/CCK, such as a combination of a gastrin and an EGF or a combination of a FACGINT and gastrin/CCK, to subjects or patients who are also treated with one or more agents that suppress the immune system, i.e., immunosuppressive agents.

A number of different endpoints can be used to determine whether gastrin and EGF or FACGINT treatment, or treatment with the combination of gastrin and EGF or FACGINT and an immunosuppressive agent, improves the diabetes, for example, increases the functional mass of β cells in the islet transplants. These include measurement of enhanced plasma levels of circulating human C peptide and human insulin, after injecting mice with β cell stimulants such as glucose or arginine; a response to gastrin/EGF treatment demonstrated by increased human insulin immunoreactivity or mRNA levels extracted from the islet transplants; and increased number of β cells, determined by morphometric measurement of islets in treated animals.

The term "transplanting" as used herein means introducing a cellular, tissue or organ composition into the body of a mammal by any method established in the art, or as indicated herein. The composition is a "transplant", and the mammal is the recipient. The transplant and recipient may be syngeneic, allogenic, or xenogeneic. The term, "autologous" as used herein means that the transplant is derived from the cells, tissues or organs of the recipient.

Enhanced β cell function of human islets can also be demonstrated by reversal of the hyperglycemia in recipient mice with streptozotocin induced or genetic (using a strain of mice known as non-obese diabetic or NOD) diabetes. Enhanced β cell function after treatment of the diabetic recipient subject with gastrin, with EGF or FACGINT and with one or more immunosuppressive agents is demonstrated by improved survival upon withdrawal of insulin, and by correcting hyperglycemia as indicated by fasting blood glucose level. Further, increases in both pancreatic insulin and plasma C-peptide are observed.

**Table 1. Exemplary agents for immune suppression, and commercial sources**

| **Name** | **Company** | **Nature** |
|---|---|---|
| 2-amino-1,3-propanediol derivatives | Novartis | Used for preventing or treating chronic rejection in a patient receiving an organ or tissue allo-or xeno-transplant |
| 2-amino-2[2-(4-octylphenyl)ethyl]propane-1,3-diol hydrochloride | Yoshitomi Pharmaceutical Industries, Ltd | Immunosuppression, from accelerated lymphocyte homing |
| 40-0-(2-hydroxyethyl)-rapamycin, SDZ-RAD, Everolimus (Certican^{®}) | Novartis Pharmaceuticals | Sirolimus (rapamycin) derivative, used for acute kidney rejection; reduces rejection and graft vasculopathy following heart transplantation by inhibiting cell proliferation |
| 6-(3-dimethyl-aminopropionyl) forskolin | Matsumori Akia Nippon Kayaju Co Ltd | Immunosuppressing action useful also for treating autoimmune disease |
| 6-mercaptopurine (Purinethol^{®}, 6-MP) | Glaxo SmithKline | Used to treat Crohn's disease, inflammatory bowel disease and for organ transplant therapy |
| ABX-CBL (CBL-1) | Abgenix | Mouse monoclonal AB targeted against human T-cell, B cells, NK cells and monocytes, fortreatment of steroid-resistant graft vs host diseases, potential use in treatment of inflammatory and autoimmune disorders |
| Alefacept (human LFA-3 IgG1 fusion protein, AMEVIVE^{®}) | University of Utah-Dermatology Dept/BIOGEN | Knocks out causative memory T-lymphocytes; Used to treat psoriasis, a T-cell mediated inflammatory disorder |
| HLA-B2702 peptide (Allotrap^{®}) | SangStat Medical | Human peptide, blocks action of NK cells and T-cell mediated toxicities, used for prevention of first kidney allograft rejection |
| Antisense ICAM-1 inhibitor (ISIS 2302), Enlimomab, BIRR1, Alicaforsen) | ISIS-Boehringer Ingleheim | Mouse monoclonal AB blocks white blood cell adhesion to T-cell surface molecule (ICAM-Ir); treatment of kidney transplant rejection |
| Azathioprine (Imuran^{®}, Azasan^{®}) | Generic, Glaxo SmithKline, Prometheus Laboratories, aaiPharma | Treatment of rheumatoid arthritis and prevention of kidney transplant rejection, and other autoimmune or inflammatory disorders such as inflammatory bowel disease |
| BTI-322 | MedImmune | Mouse derived monoclonal AB targeted to CD2 receptor; used for prevention of first-time kidney rejection, and treatment of resistant rejection |
| Cladribine (Leustatin^{®}) | Boehringer Ingleheim | Antimetabolite and immunosuppressive agent that is relatively selective for lymphocytes; used to treat lymphoid malignancies, e.g., hairy-cell leukemia. |
| Cyclophosphamide (CTX, Neosar^{®}, Cytoxan^{®}, Procytox^{®}) | Generic | Immunosuppressant t for treatment of arthritis and other auto-immune disorders and cancers |
| Cyclosporine (cyclosporin A, cyclosporin) (Sandimmune^{®}, Neoral^{®}, SangCya^{®}) | Novartis | 11 amino acid cyclic peptide; blocks helper T-cell, immunosuppressant used in organ transplant therapy and other immune diseases |
| Demethimmunomycin" (L-683,742: also described as 31-desmethoxy-31-hydroxy-L-683,590) | Merck & Co | Treatment of autoimmune diseases, infectious diseases and/or prevention of organ transplant rejections |
| Dexamethasone (Decadron, Dexone, Dexasone) | Generic | An adrenocorticoid, effective immunosuppressant in various disorders |
| Docosahexaenoic acid (DHA) | Not applicable | Immunosuppressant by that lowers the proportion of T cells expressing CD4 or CD8, blocks antigen recognition process; Taku et al., Journal of Agricultural and Food Chemistry, 2000; 48(4):1047 |
| FTY720 (oral myriocin derivative) | Novartis Pharmaceuticals | Alters lymphocyte infiltration into grafted tissues; used for prevention of organ rejection in kidney transplants |
| Glatiramer acetate (co-polymer-1, Copaxone^{®}) | Teva Pharmaceuticals | Synthetic peptide copolymer; decoy that mimics structure of myelin so immune cells bind Copaxone instead of myelin; for multiple sclerosis |
| Glial fibrillary acidic protein (GFAP) | CalBiochem; Synx Pharma | Possesses immunosuppressive activities in diabetic animal models; Winer et al., Nature Medicine 9: 198 (2003) |
| Gusperimus,(15-deoxyspergualin (Spanidin ^{®}) | Bristol Myers-Squibb | Intravenous immunosuppressant; suppresses production of cytotoxic T-cells, neutrophils and macrophages |
| hu 1124 (anti-CD11a) | XOMA | Humanized monoclonal antibody; targets CD11a receptor on surface of T cells to selectively inhibit immune system rejection of transplanted organs |
| Infliximab (Remicade^{®}) | Centocor (affiliate of Johnson and Johnson) | Monoclonal AB, binds and inactivates human TNF-alpha and ; used to treat Crohn's disease and rheumatoid arthritis |
| Interferon | Various companies including Serono, Biogen etc | Immunomodulatory properties |
| ISAtx247 | Isotechnika | Used to treat autoimmune diseases such as rheumatoid arthritis and psoriasis |
| isotretinoin | | Immunosuppressant, reduces ability of T cells to proliferate in response to immune challenge. Vergelli et al., Immunopharmacology, 1997,31:191. |
| Medi-500 (T10B9) | MedImmune | Intravenous monoclonal AB that targets human T-cells; treats acute kidney rejection and graft-vs-host disease |
| Medi-507 | MedImmune/Bio-Transplant | Intravenous humanized AB directed against CD2 T-cell; used to treat corticosteroid-resistant graft vs host disease and prevention of kidney rejection |
| Methotrexate (Rheumatrex^{®}, Amethopterin, Trexall^{®}) | Wyeth Lederle, Generic | Antimetabolite used to treat Crohn's disease, severe psoriasis, and adult rheumatoid arthritis (and as an anti-cancer drug) |
| Mitoxantrone (Novantrone^{®}) | Immunex | Antiproliferative effect on cellular immune system including T-cells, B-cells and macrophages; used to treat hormone-refractory prostate cancer, acute myelogenous leukemia and multiple sclerosis |
| mycophenolate mofetil (CellCept^{®}) | Roche | Proliferation of T and B lymphocytes by blocking the synthesis of purine nucleotides; used in organ transplant therapy and inflammatory bowel disease |
| OKT4A | R.W.Johnson Pharmaceutical Research Institute | Mouse monoclonal AB targeted against human CD4 T cell; used for prevention of kidney transplant rejection when used in combination with other immunosuppressant drugs |
| Muromonab-CD3 (Orthoclone OKT3 ^{®} )() | R.W.Johnson Pharmaceutical Research Institute | Monoclonal AB that binds to receptor sites on T-cells, preventing activation by transplanted tissue |
| Prednisolone (Deltasone^{®}, Oraone^{®}) | | Corticosteroid, suppresses inflammation associated with transplant rejection |
| basiliximab (Simulect^{®}) | Novartis Pharmaceuticals | Monoclonal AB that binds to receptor sites on T-cells, preventing activation by transplanted tissue (renal transplant) |
| S100β | glial protein | Possesses immunosuppressive activities in diabetic animal models |
| Sirolimus, Rapamycin (Rapamune^{®}) | Wyeth-Ayerst Laboratories | Immunosuppressant and potent inhibitor of cytokine (e.g.IL-2)-dependent T-cell proliferation (kidney transplant) |
| Tacrolimus (Prograf; FK-506) | Fujisawa | Interferes with IL-2 TCR communication |
| Antithymocyte immunoglobulin (ATGAM, Thymoglobulin ^{®}) | SangStat Medical Corporation, Pharmacia and Upjohn | Anti-human thymocyte immunoglobulin; used in reversal of acute kidney transplant rejection and will likely be used off-label for transplant induction therapy |
| efalizumab (Xanelim^{®}) | XOMA | T-cell modulator that target T-cells through interactions with adhesion molecules on endothelial cell surface, target migration of T-cells into the skin and target activation of T-cells; Used to treat Psoriasis |
| Daclizumab (Zenapax^{®}), HAT (Humanized Anti-Tac), SMART anti-Tac, anti-CD25, and humanized anti-IL2-receptor | Protein Design Laboratories/Roche | Monoclonal AB inhibits binding of IL-2 to IL-2 receptor by binding to IL-2 receptor; suppresses T cell activity against allografts (renal transplant) |

As used herein, the term "immunosuppressant" or "agent for immune suppression" means any agent that suppresses immune response. Exemplary immunosuppressant agents are shown in Table 1, and any derivatives of those agents or functional equivalents are considered appropriate for embodiments of the invention as described herein and in the claims.

As used herein, a dosing schedule refers to a protocol for administering any of the compositions provided herein, for example, the components that make up the LN.T.^{™} composition or the combination of a FACGINT and a gastrin/CCK receptor ligand, and one or more of an immunosuppressive agent, each in an effective dose, administered simultaneously or within a particular interval of each other, for example, within one day of eachother, or as a combined preparation, or separately, and includes the amount of the composition delivered per unit time such as per day, and the duration or period of time over which each composition is administered.

Most insulin dependent diabetic patients require insulin injection at least on a daily basis. Multiple doses per day of insulin are required under certain circumstances of illness or diet for management of diabetes, and the insulin administration is indicated by results of frequent glucose monitoring, another activity which is required of a diabetes patient for optimal management of the disease, which is performed for example as often as five times daily.

Remission from diabetes due to successful islet neogenesis therapy in combination with an immunosuppressive agent is indicated by a decreased fasting blood level of glucose, and by a decreased level and duration of elevated blood glucose in response to a dietary challenge of sugar consumption. Upon achieving successful islet neogenesis, insulin administration is reduced from, for example, five injections to two injections per day; from two injections to one injection per day; and from one to none, as indicated by data obtained from monitoring blood glucose levels. One of ordinary skill in the art of diabetology, when treating a diabetic patient, is familiar with adjusting insulin dosage to levels of blood glucose following fasting and under other physiological conditions.

Dosages of the compositions to be administered to a subject are adjusted for known variations from species to species in standard data encompassing criteria for absorption, distribution, half-life kinetics in circulation, metabolism, excretion, and toxicology of the receptor ligands of the embodiments herein, for example, for each primate and rodent species. In general, dosages are adjusted to be about 6-fold to about 100-fold greater for administration to a rodent species than to a primate species.

Immunosuppressive agents in Table 1 or other equivalent agents are administered as supplied by the manufacturers, normalizing to body weight of the subject as is known by one of skill in the pharmacological arts. For example, Tacrolimus is generally administered by injection or orally, and Sirolimus is generally administered orally.

Modes of administration of the receptor ligand compositions and immunosuppressive agents include but are not limited to subcutaneous, transdermal, intrathecal, intramuscular, intraperitoneal, intravenous, intranasal, and oral routes. The compounds may be administered by any convenient route, for example, by infusion or bolus injection, by pump, by absorption through epithelial or mucocutaneous linings (e.g., oral mucosa, rectal and intestinal mucosa, etc.). The receptor ligands herein may be administered in combination with one or a plurality of other biologically active agents. For example, a recipient of the compositions and methods herein may be administered one or more antibiotics if a bacterial infection is present, or aspirin if a headache is present. Administration of the receptor ligands herein is preferably by a systemic route.

The invention in one embodiment provides a method for treating diabetes mellitus by administering a composition comprising both a gastrin/CCK receptor ligand, e.g. gastrin, and an EGF receptor ligand, or a FACGINT, e.g. GLP-1, GH, or prolactin in a formulation for sustained release, to effect differentiation of pancreatic islet precursor cells to mature insulin-secreting cells. Both the FACGINT and the gastrin/CCK receptor ligand in the composition can be administered systemically or locally.

Without being limited by any particular mechanism, prolonged efficacious islet cell neogenesis is achieved following administration of both a gastrin/CCK receptor ligand, such as gastrin, and an EGF receptor ligand or one or more of a member of the FACGINT group as defined herein, such as a GLP-1, a GH, or a prolactin, alone or with an agent for immune suppresion, and any one or more of these receptor ligands or factors or agents being formulated for sustained release as described herein or by equivalent methods known or one of ordinary skill in the pharmacological arts.

The invention in a general embodiment provides a method for preventing or treating diabetes, the method comprising administering to a mammal in need thereof a composition comprising a sustained release formulation of at least one of an EGF receptor ligand or a FACGINT, such as a GLP-1, an exending-4, a growth hormone, a prolactin, in combination with a gastrin/CCK receptor ligand, each of the EGF receptor ligand or the FACGINT and the gastrin/CCK receptor ligand in an amount sufficient to increase the number of pancreatic insulin secreting β cells in the mammal, thereby treating or preventing the diabetes. Compositions for treating diabetic subjects and patients with a sustained release formulation of a FACGINT comprise : a PTH-related protein (PTHrP) receptor ligand such as PTHrP (PTHrP; Garcia-Ocana, A. et al., 2001, J. clin. Endocrin. Metab. 86: 984-988); a hepatocyte growth factor (HGF) receptor ligand such as HGF (HGF; Nielsen, J. et al., 1999, J Mol Med 77: 62-66); a fibroblast growth factor (FGF) such as FGF, a keratinocyte growth factor (KGF) receptor ligand such as KGF; a nerve growth factor (NGF) receptor ligand such as NGF; a gastric inhibitory polypeptide (GIP) receptor such as GIP; a transforming growth factor beta (TGFβ) receptor ligand such as TGFβ (U.S. patent application 2002/0072115 published Jun. 13, 2002), a laminin receptor ligand such as laminin-1; an islet neogenesis associated protein (INGAP) receptor ligand such as INGAP; a bone morphogenetic factor (BMP) receptor ligand such as BMP-2; a vasoactive intestinal peptide (VIP) receptor ligand such as VIP; a glucagon-like peptide 1 receptor ligand such as GLP-1 and exendin-4, glucagon-like peptide 2 (GLP-2) receptor ligand such as GLP-2, and dipeptidyl peptidase IV inhibitors which indirectly affect the levels of GLP-1 (Hughes, T. et al., 2002, Am Diabetes Assoc Abstract 272-or) by inhibiting an enzyme involved in its integrity; a REG receptor ligand such as REG protein; a Growth hormone (GH) receptor ligand such a GH, a Prolactin (PRL) receptor ligand such as PRL and placental lactogen (PL); an Insulin-like growth factor (Type 1 and 2) receptor ligands such as IGF1 and IGF-2; an Erythropoietin (EPO) receptor ligand such as EPO (http://www.drinet.org/html/august_2002_.htm); a betacellulin (also considered to be a member of the EGF family); an Activin-A receptor ligand such as Activin-A; a vascular Activin-A; a vascular endothelial growth factor (VEGF) receptor ligand such as VEGF; a bone morphogenesis factor (BMP) receptor ligand such as BMP-2; a vasoactive intestinal peptide (VIP) receptor ligand such as VIP; a vascular endothelial growth factor (VEGF) receptor ligand such as VEGF; a pituitary adenylate cyclase activating polypeptide (PACAP) receptor ligand such as PACAP; a granulocyte colony stimulating factor (G-CSF) receptor ligand such as G-CSF; a granulocyte-macrophage colony stimulating factor (GM-CSF) receptor ligand such as GM-CSH; a platelet-derived growth factor (PDGF) receptor ligand such as PDGF and a Secretin receptor ligand such as secretin. These sustained release formulations may also include an agent for immune suppression.

In one embodiment, the sustained release formulation of the composition is administered systemically. Alternatively, the composition is administered locally, or with a device or means for local delivery. The mammal is a diabetic mammal, for example, the mammal has been diabetic for an extent of at least about 1% of the lifespan of the mammal. In general, the amount of the sustained release formulation of the gastrin/CCK receptor ligand, the FACGINT or the EGF receptor ligand in the composition is substantially lower than the minimum effective dose of any of these alone required to reduce blood glucose in the diabetic mammal. The FACGINT or the EGF receptor ligand and the gastrin/CCK receptor ligand are provided in amounts sufficient in combination to induce differentiation of the pancreatic islet precursor cells into glucose responsive insulin secreting islet cells for a prolonged period of time.

Another embodiment of the invention provides a method for preventing or treating diabetes, the method comprising administering to a mammal in need thereof a sustained release formulation of a composition comprising a combination of a FACGINT or an EGF receptor ligand, and a gastrin/CCK receptor ligand, in an amount sufficient to increase proliferation of islet precursor cells in pancreatic tissue, thereby treating or preventing the diabetes.

In another aspect, the invention provides a method for preventing or treating diabetes, the method comprising administering to a mammal in need thereof a sustained release formulation of a composition comprising a combination of a FACGINT or an EGF receptor ligand, and a gastrin/CCK receptor ligand, each in an amount sufficient to increase the number of pancreatic insulin secreting β cells in the mammal; and determining the amount of islet neogenesis, thereby treating or preventing the diabetes. Administering the composition reduces blood glucose compared to blood glucose assayed prior to administering the composition, for example, administering the composition reduces blood glucose by about 50%, or by about 70%, compared to blood glucose assayed prior to administering the composition. Glycosylated hemoglobin concentration is reduced compared to glycosylated hemoglobin concentration in the mammal assayed prior to administering the composition. Serum insulin concentration is increased compared to serum insulin concentration in the mammal assayed prior to administering the composition. Pancreatic insulin concentration is increased compared to pancreatic insulin concentration in the mammal assayed prior to administering the composition.

In another aspect, the invention provides a method for inducing pancreatic islet neogenesis in a mammal, the method comprising administering to the mammal a sustained release formulation of a composition comprising a combination of a FACGINT or an EGF receptor ligand and a gastrin/CCK receptor ligand, each in an amount sufficient to increase proliferation of islet precursor cells in pancreatic tissue, thereby inducing pancreatic islet neogenesis.

In another aspect, the invention provides a method for inducing pancreatic islet neogenesis in a mammal, the method comprising administering a composition comprising a sustained release formulation of a combination of a FACGINT or an EGF receptor ligand and a gastrin/CCK receptor ligand, each in an amount sufficient to increase the number of pancreatic insulin secreting β cells in the mammal.

In another aspect, the invention features a composition comprising a gastrin/CCK receptor ligand, and a FACGINT or an EGF receptor ligand, any of which agents are formulated in sustained release formulation. The composition is in a dosage effective for inducing proliferation of islet precursor cells into an increased amount of mature insulin secreting cells. Further, the composition is in a dosage effective for inducing differentiation of an islet precursor cell into a mature insulin secreting cell. The composition can be in a pharmaceutically acceptable carrier. The composition can include an agent for suppression of an immune response.

In another aspect, the invention provides a kit for treating or preventing diabetes, containing a composition comprising a gastrin/CCK receptor ligand and a FACGINT or an EGF receptor ligand, any of which are present in sustained release formulation, a container, and instructions for use. The composition can include an agent for immune suppression. The composition of the kit can further comprise a pharmaceutically acceptable carrier. The composition of the kit can be present in a unit dosage.

Another embodiment of the invention provided herein is a method for expanding and differentiating stem cells in a diabetic recipient of the cells into insulin secreting cells, comprising implanting the cells in the recipient, and administering a composition containing an effective dose of each of a gastrin/CCK receptor ligand and a FACGINT or an EGF receptor ligand, one or more of which are present as a sustained release formulation. For example, the implanted cells are obtained from a human. Further, the implanted cells are obtained from pancreatic islets, umbilical chords, embryos, or stem cell lines. Generally, the gastrin/CCK receptor ligand is human gastrin 1-17Leu15. Generally, the EGF receptor ligand is an EGF or a TGFα, or is a polypeptide that structurally is substantially identical to EGF or TGFα, respectively, and has substantially the same biological functions of each of EGF or TGFα. In a related embodiment, cells, for example, stem cells are implanted by a route selected from: direct injection into an organ, and by intravenous administration. For example, the cells are injected into an organ selected from the pancreas, the kidney, and the liver. Alternatively, the cells are administered to the portal vein using a percutaneous or transhepatic route. In either case, prior to implanting, the cells can be treated ex vivo with the composition.

Another embodiment of the invention provided herein is a method of treating human diabetes by implanting a reduced amount of stem cells into a diabetic recipient, the method comprising administering to the recipient an effective dose of each of a sustained release formulation of a gastrin/CCK receptor ligand and a FACGINT or an EGF receptor ligand, the amount of cells reduced in comparison to implanting cells into an otherwise identical recipient in the absence of administering the effective dose. The recipient can further be administered an agent that suppresses the immune system. For example, the agent is a chemical selected from the group consisting of FK506, rapamycin, cyclosporin and cortisone. Alternatively, the agent is an antibody, for example, the antibody is anti-CD4. In any of the methods provided that involve stem cells, the cells prior to implanting can be obtained from a family member and stored for later use.

An embodiment of the present invention provides improved methods and compositions for use of a FACGINT or an EGF receptor ligand administered with a gastrin/CCK ligand, any one or more of these agents being formulated for sustained release, to treat diabetes. The present invention in one embodiment provides a sustained release formulation of any of: a gastrin, in combination with a FACGINT or an EGF receptor ligand, to achieve greater efficacy, potency, and utility than achieved with a FACGINT or an EGF receptor ligand alone, or with a combination of any of these agents administered to provide immediate bioavailability of the entire formulation. The present invention provides an improved therapeutic ratio for the sustained release formulation compared to the immediately bioavailable formulation.

Use of a sustained release formulation can extend the reduction in blood sugar for an even greater period of time.

The combination of a gastrin/CCK receptor ligand and an EGF receptor ligand or FACGINT in a sustained release formulation, with systemic administration, had greater potency than when it was administered in a non-sustained release direct formulation. An improvement in glucose tolerance and an increase in pancreatic insulin levels were observed with sustained release formulation of the gastrin/FACGINT combination or gastrin/EGF receptor ligand combination.

The method for treating diabetes mellitus in an individual in need thereof includes administering to the individual a sustained release formulation of a composition that provides both a gastrin/CCK receptor ligand and a FACGINT, or gastrin/CCK receptor ligand and an EGF receptor ligand, the compositions in doses sufficient to effect differentiation of pancreatic islet precursor cells to mature insulin-secreting cells. The cells differentiated are residual latent islet precursor cells in the pancreatic duct. A method for treating insulin dependent diabetes, especially Type I or juvenile diabetes mellitus, comprises administering, preferably systemically, a differentiation regenerative amount of both a gastrin/CCK receptor ligand and a FACGINT or a gastrin/CCK receptor ligand and an EGF receptor ligand, either or both agents in a sustained release formulation, to a diabetic mammal, to stimulate islet neogenesis to increase the number of functional glucose responsive insulin secreting β cells in the pancreas. The combination of a gastrin and either a FACGINT or an EGF receptor ligand, any of which in a sustained release formulation, would result in a significant enhancement of the islet neogenesis response over that observed with the same agents but in a non-sustained release direct formulation. An exemplary gastrin/CCK receptor ligand is gastrin or its synthetic gastrin derivative as described herein, and exemplary FACGINTs are GLP-1, GH, and prolactin. An exemplary EGF receptor ligand is a recombinant human EGF, for example, EGF51N, a 51 amino acid long human recombinant mutant EGF having a deletion at the C-terminal and an asparagine residue at position 51.

Alternatively, the cells are administered to the portal vein using a percutaneous or transhepatic route. In either case, prior to implanting, the cells can be treated ex vivo with the composition.

### Formulations for Sustained Release and Local Delivery and Methods of Administration

Administration of at least one of the agents that are a gastrin/CCK receptor ligand, or an EGF receptor ligand or a FACGINT, is formulated to occur by sustained or controlled release. "Sustained release" as used here and in the claims refers to a combination of materials, devices, formulation, and/or administration of at least one I.N.T.^{™} therapeutic agent, that creates a continuous or discontinuous, such as cyclical, supply of an amount of the combination or at least one I.N.T. agent, or immune suppressing agent, to the recipient as a function of time. The period of time over which release can be sustained is minutes, hours, days, weeks, or even months. The release of the active agent may be constant over a long period, alternatively release may be cyclical over the prolonged period release. Release may be independent of conditions, alternatively release may be triggered in response to a composition in the environment or to other external events. For example, release may be triggered by an endogenous composition such as insulin or glucose, or release may be triggered by an externally supplied composition such as in response to administration of a drug.

Sustained release is contrasted to a non-sustained release formulation that delivers the whole of the therapeutic agent dose instantaneously or very rapidly, or is bioavailable in the entirety of the composition in a very short period following administration, making a large proportion of the agent available to the recipient over a very short time period. Examples of substantially instantaneous bioavailability of a dose include aqueous solutions of agents that are administered parenterally, for example by a bolus intraperitoneal injection, or orally, or even administered by intravenous drip over a period of several hours. Thus, intravenous administration of a cardiovascular imaging agent is circulated throughout the arterial system within 15 seconds; intravenous drip administration of an anti-tumor agent is entirely bioavailable within seconds of finishing the drip. In contrast, sustained release formulations benefit the recipient patient both by providing long term bioavailability, and by protein the patient by avoiding potential side effects that might result from initial high levels of the agent. See Mathiowitz, E., Encyclopedia of Controlled Drug Delivery. 1999. New York: John Wiley.

Sustained release formulations have been developed to provide systemic as well as local (or targeted) administration of agents. A variety of materials, devices and routes of administration reviewed herein is used with the I.N.T.^{™} agent such as the gastrin/CCK receptor ligand, and the EGF receptor ligand or the FACGINT.

Oral sustained release systems have been available, for example, hard gelatin capsules containing a plurality of types of pellets, each type having a different thickness of coating such that some pellets are uncoated more quickly. See Banga, A., Bus. Brief.: Pharmatech 2002: 151-154. In oral osmotic systems, fluid entering a tablet through a semi-permeable membrane generates an osmotic pressure of core components that is independent of variables in the gastrointestinal tract. To protect agents that are macromolecules, for example proteins such as growth factors, methods include use of site-specific delivery, protease inhibitors, carrier systems, or formulations such as hydrogels that contain polyacrylic acid backbones and bioadhesive properties.

Most protein agents are delivered parenterally, and controlled release methods for parenteral delivery of proteins include use of lactic acid-based polymers such as poly(D, L-lactide-co-glycolide; PLGA), which forms biodegradable microspheres with a core containing the agent, and that release the agent over a course of time of about one month. Further, proteins or liposomes (see below) containing protein can be pegylated by covalent addition of polyetheylene glycol (PEG).

Transdermal delivery can be used both for systemic and for local delivery. Permeation enhancers as described infra can be used in combination with a transdermal patch to improve delivery, or in combination with a device for iontophoresis, phonophoresis, microporation, or elecroporation with possible wearable electrical devices.

The term, "local delivery" as used herein refers to administering by a route and formulation or device or both such that a particular target organ or tissue is substantially treated while other organs and tissues are not so treated, or that the extent of treatment of the target tissue or organ receives at least two-fold, at least five-fold, or at least 10-fold greater dose than non-target tissues or organs. In general herein, a target organ is the pancreas. As shown herein, cells for transplantation or multi-cellular transplants can be locally delivered to the pancreas by injection into the portal vein; drugs can be delivered by injection into pancreatic arteries, hepatic arteries, portal vein, or pancreatic duct. It is possible to employ a pump that is not implanted, i.e., remains external and delivers the composition to a target organ such as the pancreas by a catheter connecting the pump to the organ. It is also possible to employ an implantable pump to deliver the composition locally.

Other procedures for local delivery include without limitation endoscopic retrograde cholangiopancreatography (ERCP); endoscopic ultrasound-guided fine needle aspiration biopsy (EUS-FNAB) which is adapted for delivery of pharmaceutical compositions provided herein, rather than used for sampling. See for example, Wang et al., Transpl. Int 1995, 8: 268-272. While these technologies are devised for diagnostic or prognostic purposes, they can be adapted for delivery of compositions herein, which may LN.T.^{™} compositions as described herein, to be combined with immune suppressing agents, and or as sustained release formulations. See also, Yano et al., 1994, Transpl Int 7 Suppl 1:S187-193; Ricordi et al., 1994, Transpl Proc 26: 3479; and Munoz-Acedo et al., 1995, J Endocrin 145: 227-234.

Pumps which can be implantable or non-implantable (external) pumps for drug delivery are in use for treatment of several diseases, for example, for cancer and for diabetes. A pump can be a peristaltic pump, a fluorocarbon propellant pump, or an osmotic pump including a mini-osmotic pump (Blanchard, S., 1996 Biomedical Engineering Applications, North Carolina State University). Peristaltic pumps deliver a set amount of drug with each electric pulse that drives the pump head. The pump, electronics and power source are located in a titanium housing covered in Silastic. Drug reservoirs are silicone rubber pouches that can withstand substantial pressure, for example, 60 psi. The reservoir can be refilled percutaneously through a polypropylene port. Fluorocarbon pumps use a fluorocarbon liquid to operate the pump. Osmotic pumps use osmotic pressure to release the drug at a constant rate. An exemplary pump is the MiniMed MicroMed 407C pump (Medtronic, Inc., Northridge, CA). Further, an intrathecal drug delivery system (Medronic) which includes two implantable components, an infusion pump, and an intraspinal catheter, can be used. The pump is inserted abdominally in a subcutaneous pocket, while the catheter is inserted into the intrathecal space of the spine, tunneled under the skin, and connected to the pump. Medication is then delivered at a constant or variable flow rate. Further, an intraperitoneal pump, for instance an implantable pump, can be used to deliver the compositions herein locally, for example via a catheter, or systemically.

Mucosal delivery to epithelial areas that are maintained in a moist condition and which have close underlying vasculature can be more efficient than transdermal delivery across a tissue which is a dry epidermal surface. Mucosal surfaces include: nasal, pulmonary, rectal, buccal, ocular, and genital. Exemplary mucosal surfaces are nasal and pulmonary.

Materials used for microspheres for sustained release are primarily polymers, and include PEG, also called polyethylene oxide (PEO), and PGLA as described. A polymer vehicle can be injected directly, allowing for gradual hydrolysis or degradation within the subject to release the therapeutic agent. The molecular weight of the polymer, e.g., PEG, can be varied to control the release rate. Pegylation or covalent attachment of PEG to a protein therapeutic agent shields the protein from receptors involved in clearance mechanisms such as that of the reticulo-endothelial system (RES). Alternatively, polysaccharides can be used to target an agent to the RES (U.S. patent 5, 554,386 issued Sept. 10, 1996). Organs of the RES include liver, spleen, and bone marrow.

Homopolymers or co-polymers, such as poly(lactic acid-co-ethylene glycol) or PLA-PEG, can form a viscous liquid which is mixed with a therapeutic protein agent. Viscosity is varied by the molecular weight of the PLA-PEG. Under certain conditions the therapeutic agent co-precipitates with the polymer upon injection into the subject and loss of the solvent by diffusion, such that a depot is formed having favorable release kinetics (Whitaker, M., et al. Bus. Brief: Pharmatech 2002:1-5).

Microparticles are formed of polymeric microspheres that encapsulate the therapeutic agent. Polymers for use in microspheres include poly(lactic acid) or PLA; poly(glycolic acid) or PGA; and copolymer PLA-PGA. An amount of the therapeutic agent such as the proteins of the present invention, is released in stages such as an initial burst of protein non-specifically associated with the exterior of the particles, a later stage by diffusion, and a final stage by erosion can be controlled by polymer composition, molecular weight, size of the microparticles, and physiological conditions such as pH. Protein stability during manufacture of microspheres is enhanced if the protein is in solid form, such as a lyophilized powder, which is emulsified with solvent or by a frozen-atomization process or a sonication process, and the suspension is then frozen in liquid nitrogen for further solvent extraction. Microspheres can be produced from a supercritical fluid, e.g., supercritical carbon dioxide (scCO₂).

Biodegradable block polymers that are suitable for drug delivery and methods of synthesis are described by Kumar, N. et al., Adv. Drug Deliv. Rev. 53 (2001): 23-44. Copolymers can be random, alternating, or block (di or tri type) and can be linear, or star or graft (comb-shaped) in configuration. A polymer can form a hydrogel, which is a three-dimensional, hydrophilic polymeric network that holds a large amount of aqueous fluid. The polymer used in a hydrogel is rendered insoluble by cross-linking or other chemical adducts.

Biodegradable implants can be prepared from materials such as at least one of the materials selected from the group of: starch; vinylstarch; dipropyleneglycol diacrylate (DPGDA); tripropyleneglycol diacrylate (TPGDA); pectin; cellulose acetate; cellulose propionate; cellulose acetate butyrate; cellulose acetate propionate (CAP); hydroxypropyl cellulose (HPC); hydroxypropyl cellulose/cellulose acetate propionate (HPC/CAP); methyl methacrylate (MMA); butyl methacrylate (BMA); hydroxymethyl methacrylate (HEMA); ethyl hexyl acrylate (EHA); octadecyl methacrylate (ODMA); and ethyleneglycol dimethacrylate (EGDMA). See Gil, M. et al., Boletim de Biotecnologia 2002, 72:13-19.

In addition to polymers, naturally occurring and synthetic lipids can be used for sustained release formulations. DepoFoam™ (Skye Pharma, London, England) forms a multivesicular lipid-based particle (liposome) for encapsulating therapeutic agents (see U.S. Patent 5,993,850; and Ye, Q. et al., 2000 J. Controlled Rel. 64:155-166). The lipids are amphipathic with a net negative charge, sterols, or zwitterionic lipids, and methods for making the liposomes are non-acidic. Methods for incorporating an active therapeutic agent into the liposomes are also provided. The therapeutic agent can be one or more of a gastrin/CCK receptor ligand, an EGF receptor ligand, and a FACGINT.

Other lipids for liposomes are within the scope of the invention. A plant polar lipid, for example a ceramide such as a wheat ceramide, is useful for forming a gel with a protein such as a prolamine, into which one or more therapeutic agents can be placed for transdermal or transmucosal delivery. See U.S. Patent 6,410,048, issued June 25, 2002. Exemplary prolamines include wheat gliadin, and corn zein. Other naturally occurring polymers used in sustained release drug formulations and devices include collagen (EP-A-O 621 044), chitin (U.S. patent 4,393,373), and chitosan, a deacylated form of chitin.

A permeation enhancer, for example, a glycolipid, a non-esterified fatty acid, an aliphatic alcohol, a fatty acid ester of an aliphatic alcohol, a cyclohexanol, a fatty acid, ester of glycerol, a glycol, or an aliphatic alcohol ether or a glycol, are typical permeation enhancers, other components such as a stabilizer, a solubilizer, a surfactant and a plasticizer can be present in a transdermal device. See U.S. patent application 20020127254, published September 12, 2002.

Lipids and a variety of types of polymers are used to form "nanoparticles" for drug delivery, reviewed by M. Kumar, 2002, J. Pharm. Pharmaceut. Sci. 3:234-258. Drug loading into these particles is found to be greatest with most lipophilic therapeutic agents. Long lasting release has been found with liposomes comprising polylactic acid, lecithin, and phosphatidylcholine or cholesterol.

Local (targeted) sustained release is obtained by methods described herein, for example, use of liposomes comprised of a material designed to target the RES, or comprised of a different material to avoid cells of the RES. Additional targeting methods include use of antibodies or soluble recombinant receptors in conjunction with the outer surface of liposomes or microspheres. Further, sustained release formulations as described herein may be further adjusted for use with any of the devices described herein, such as a pump, for local delivery to a particular target organ.

The present invention in another embodiment provides sustained release pharmaceutical compositions comprising a therapeutically effective amount of a combination of a FACGINT or an EGF receptor ligand, and a gastrin/CCK receptor ligand. A pharmaceutically acceptable carrier or excipient can be added. Such a carrier includes but is not limited to saline, buffered saline, dextrose, water, glycerol, ethanol, and combinations thereof. The formulation should suit the mode of administration.

Unless otherwise defined, all technical and scientific terms herein have the same meaning as commonly understood by one of ordinary skill in the art to which this invention pertains. Methods and materials similar or equivalent to those described herein can be used in the practice of the present invention. The invention in various embodiments now having been fully described, additional embodiments are exemplified by the following Examples and claims, which are not intended to be construed as further limiting. The contents of all cited references are hereby incorporated by reference in their entirety herein.

### EXAMPLES

### Example 1. Treatment with a GLP-1 receptor ligand glucaaons-like peptide 1 (GLP-1), and a gastrin/CCK receptor ligand, gastrin, prevents disease progression in NOD mice with recent-onset diabetes.

Mice of the non-obese diabetic (NOD) strain have a phenotype that shares many features of disease pathogenesis with human type I diabetes. NOD mice typically exhibit destructive autoimmune pancreatic insulitis and β-cell destruction as early as four weeks of age. Diabetes onset usually occurs at age 10-15 weeks in these mice, with typical blood glucose levels observed to be between about 7mM to about 10 mM (compared to a range of about 3.0-6.6 mM normal mice), and a pancreatic insulin level that is lower by more than about 95% than that in normal mice. As disease progresses, NOD mice exhibit increasingly severe signs of chronic diabetes, with blood glucose levels reaching between about 25 to about 30 mM, and pancreatic insulin level declining to become virtually non-existent. At that severe stage of the disease, greater than about 99% of β-cells have been destroyed.

In this example, the effect of treatment by a combination of GLP-1 and gastrin was examined in NOD mice with recent onset diabetes, to determine whether administration of both GLP-1 and gastrin would prevent severe hyperglycemia, ketoacidosis and death as well as increase pancreatic insulin content in NOD mice with recent-onset diabetes. The LN.T.^{™} composition used was gastrin as synthetic human gastrin I having 17 amino acid residues with a Leu residue at amino acid position 15. The GLP-1 used was GLP-1 which is the biologically active fragment of human/mouse GLP-1 (having residues at positions 7-36 compared to the precursor from which the fragment is processed; obtained from Bachem H6795).

Non-obese diabetic (NOD) female mice, ages 12-14 weeks, were monitored for development of onset of diabetes (fasting blood glucose > 8.0 to 15 mmol/l), and within 48 hours after onset of symptoms, two groups of mice were each treated as follows: one group was treated with vehicle only; and the other group was administered 100 µg/kg/day of GLP-1, each treatment administered via the intraperitoneal route twice daily.

Therapy was administered for 14 days. Animals were monitored weekly for fasting blood glucose (FBG) levels. FBG levels were measured at about 12 hours after food had been withdrawn, and 24 hours after the last peptide or vehicle injection. Upon cessation of therapy, all mice were monitored for FBG levels for the next 4 weeks (weeks 2-6) so as to determine whether prevention of hyperglycemia persisted after termination of therapeutic treatment. At 14 days treatment was stopped, and at 18 days the mice were sampled to obtain FBG levels (as shown in Table 2).

**Table 2. Glucagon-like peptide -1 (GLP-1) and gastrin combination therapy treats recent onset diabetes in NOD mice**

| Group | GLP-1 | Gastrin | Number | | FBG (mM) at day: | | |
|---|---|---|---|---|---|---|---|
| | | | | 0 | 7 | 14 | 18 |
| 1 | - | - | 6 | 11.0±0.6 | 14.8±1.3 | 22.8±1.6 | 24.4±1.5 |
| 2 | + | - | 4 | 12.3±0.9 | 14.1±1.8 | 15.3±2.6 | 15.8±4.2 |
| 3 | - | + | 4 | 11.8±0.9 | 14.8±3.4 | 16.4±3.4 | 19.0± 4.5 |
| 4 | + | + | 4 | 13.5±0.9 | 10.4±0.4 | 7.9±0.8 | 7.9±1.5 |

| | | | | | | | |
|---|---|---|---|---|---|---|---|
| GLP- 1 (100 µg/kg/day) and gastrin (3 µg/kg/day) were administered by i.p. injection to NOD female mice, twice a day, to each group as shown above. Diabetic mice , aged 12-14 weeks, were used within two days of diabetes onset (generally considered to be an FBG of more than 6.5 mM). | | | | | | | |

The protocol includes sampling of these mice for data again at 6 weeks, and blood collected for assay of FBG and plasma C-peptide, and the mice are sacrificed for pancreatic insulin determinations and scoring of islet inflammation (insulitis). From the outset of treatment, mice received neither insulin-replacement treatment nor immunosuppression. The following parameters are assessed: survival rates, pancreatic insulin levels, presence of islet inflammation and fasting blood glucose levels.

Results show that in animals of the vehicle-treated control group (group 1), fasting blood glucose (FBG) values increased progressively during the time course of this mock treatment, from 11 mM glucose at day 0 to 24 mM at day 18.

In contrast, in mice in treated with GLP-1 and gastrin, FBG values (7.9 mM glucose) were significantly less compared to the vehicle-treated mice (24.4 mM), reduced to a level, in fact, that is less than one third of the level that developed in the vehicle trated mice; Table 2). Most significant and surprisingly, the combination of GLP-1 and gastrin was more effective in decreasing FBG levels than either gastrin alone or GLP-1 alone (having FBG levies of 19.0 mM and 15.8 mM, respectively). Only in mice treated with the combination was the FBG reduced to a level that is in a normal range. The improved control of blood glucose levels in mice treated with GLP-1 and gastrin may be expected to be associated with a significantly increased content of insulin in the pancreas of these mice.

In summary, the results in this study show that treatment with a short course of low doses of GLP-1 and gastrin treatment in mice with recent onset of diabetes prevented disease progression, and reversed the disease condition to yield a blood glucose level of about normal. Further, this significant decreased blood glucose level in mice treated with gastrin and GLP-1 persisted for an additional time period after cessation of treatment. It is expected that in these animals, data will show improved pancreatic insulin content, and that these effects will be shown to be sustained for a prolonged period of time after termination of therapy.

Further groups of NOD female mice were treated with a prolactin receptor ligand, prolactin (PRL), and a gastrin/CCK receptor ligand, and with a growth hormone receptor ligand, growth hormone (GH), and a gastrin/CCK receptor ligand, gastrin. It is anticipated that data will indicate that these treatments yield effects on FBG and other parameters that are similar to data obtained herein with GLP-1.

### Example 2. Comparison of compositions with and without Pegylation

In order to determine whether a sustained release formulation would provide greater efficacy than an otherwise identical formulation that is formulated for bolus or non-sustained release delivery, a study is performd comparing a protocol of treating NOD mice with an LN.T.™ composition either in a pegylated or non-pegylated formulation. Pegylation of at least one component of the LN.T.™ composition can prolong the amount of time the therapeutic agent is retained ina na active form in the body.

The study shows that administration of a sustained release formulation of at least one agent of an LN.T.™ composition is able to improve the diabetic conditions of the NOD mice, as compared to an LN.T.™ composition formulated for direct, i.e., non-sustained release administration.

### Example 3. Comparison of frequency of dosages composition administration

In order to determine whether a sustained release formulation would provide greater efficacy than a non-sustained release direct formulation, an experiment is devised having comparing a protocol of three-time a day administration to the once-α-day administration of an I.N.T.^{™} composition to NOD mice.

The results show that prolongation of bioavailability of the I.N.T.™ composition, comparable to that obtained with a sustained release formulation of an I.N.T composition, can improve efficacy, i.e., can better remediate symptoms of the diabetic condition of the NOD mice, as compared to a direct or non-sustained release formulation of an LN.T.^{™} composition, and can reduce the frequency of dosages required for such remediation of symptoms.

## Claims

1. A gastrin/CCK receptor ligand for use in separate, simultaneous or sequential combination with a GLP-1 receptor ligand in the treatment of diabetes.

2. The gastrin/CCK receptor ligand according to claim 1 for use in separate, simultaneous or sequential combination with a GLP-1 receptor ligand, wherein the GLP-1 receptor ligand is exendin-4.

3. The gastrin/CCK receptor ligand according to claim 2 for use in separate, simultaneous or sequential combination with a GLP-1 receptor ligand, wherein the GLP-1 receptor ligand is exenatide.

4. The gastrin/CCK receptor ligand according to claim 1 for use in separate, simultaneous or sequential combination with a GLP-1 receptor ligand, wherein at least one of the gastrin/CCK receptor ligand or the GLP-1 receptor ligand is formulated for sustained release administration.

5. The gastrin/CCK receptor ligand according to any one of the preceding claims, wherein the gastrin/CCK receptor ligand comprises the carboxy terminal amino acid sequence Trp-Met-Asp-Phe-amide.

6. The gastrin/CCK receptor ligand according to claim 5, wherein the gastrin/CCK receptor ligand is a gastrin selected from: gastrin 8, gastrin 17 and gastrin 34.

7. The gastrin/CCK receptor ligand according to claim 6, wherein the gastrin is gastrin 17.

8. The gastrin/CCK receptor ligand according to claim 7, wherein the gastrin 17 is human gastrin 1-17Leu15.

9. The gastrin/CCK receptor ligand according to any one of the preceding claims for use in separate, simultaneous or sequential combination with a GLP-1 receptor ligand, wherein the use further comprises the administration of an agent for suppressing an immune response.

10. The gastrin/CCK receptor ligand according to claim 9 for use in separate, simultaneous or sequential combination with a GLP-1 receptor ligand, wherein the agent for suppressing an immune response is a drug.

11. The gastrin/CCK receptor ligand according to claim 10 for use in separate, simultaneous or sequential combination with a GLP-1 receptor ligand, wherein the drug is at least one of the group consisting of a rapamycin, a corticosteroid, an azathioprine, mycophenolate mofetil, a cyclosporine, a cyclophosphamide, a methotrexate, a 6-mercaptopurine, FK506, 15-deoxyspergualin, an FTY 720, a mitoxantrone, a 2-amino-1,3-propanediol, a 2-amino-2[2-(4-octylphenyl)ethyl]propane-1,3-diol hydrochloride, a 6-(3-dimethyl-aminoproionyl) forskolin and a demethimmunomycin.

12. The gastrin/CCK receptor ligand according to claim 9 for use in separate, simultaneous or sequential combination with a GLP-1 receptor ligand, wherein the agent for suppressing an immune response is a protein.

13. The gastrin/CCK receptor ligand according to claim 12 for use in separate, simultaneous or sequential combination with a GLP-1 receptor ligand, wherein the protein comprises an amino acid sequence of an antibody.

14. The gastrin/CCK receptor ligand according to claim 13 for use in separate, simultaneous or sequential combination with a GLP-1 receptor ligand, wherein the antibody is hul 124, BTI-322, allotrap-HLA-B270, OKT4A, Enlimomab, ABX-CBL, OKT3, ATGAM, basiliximab, daclizumab, thymoglobulin, ISAtx247, Medi-500, Medi-507, Alefacept, efalizumab, infliximab, and an interferon.

15. A GLP-1 receptor ligand for use in separate, simultaneous or sequential combination with a gastrin/CCK receptor ligand in the treatment of diabetes.
